# EUROPEAN PATENT SPECIFICATION

(11) **EP 3 773 871 B1**
(45) Date of publication and mention of the grant of the patent: **14.12.2022**
(21) Application number: 18722815.0
(22) Date of filing: 13.04.2018
(51) Int. Cl.: A61N 1/05

(54) **PULL WIRE DISPLACEMENT ASSEMBLIES**
ANORDNUNGEN ZUM BEWEGEN VON ZUGDRÄHTEN
ENSEMBLES DE DÉPLACEMENT DE FIL DE TRACTION

(43) Date of publication of application: 17.02.2021
(73) Proprietor: Advanced Bionics AG, 8712 Staefa (CH)
(72) Inventor: PATNALA, Anil K., Stevenson Ranch, California 91381 (US)
(74) Representative: Schwan Schorer & Partner mbB
(86) International application number: PCT/US2018/027626
(87) International publication number: WO 2019/199333

(56) References cited:
- WO-A1-98/40119
- WO-A1-2010/005627
- US-A- 5 861 024
- US-A- 6 156 027
- US-A1- 2011 319 909

## Description

### BACKGROUND INFORMATION

Pull wires may be coupled to various types of leads, catheters, stylets, and other elongate tubes and instruments configured to be inserted into the human body as part of various types of surgical operations and/or other medical procedures. As such, pull wires coupled to such tubes and instruments may make it possible to control distal portions of the tubes and instruments, which may be located internal to a patient during a medical procedure, by manipulating (e.g., pulling, releasing, etc.) the pull wires externally to the patient.

One exemplary context in which pull wires may be useful is the surgical implanting of an electrode lead within a cochlea of a patient as part of an initial implantation and setup of a cochlear implant system. The surgical procedure by way of which the electrode lead is inserted into the cochlea (referred to herein as an "electrode lead insertion procedure" or an "insertion procedure") may be a delicate and difficult procedure to perform. Even when performed with great care and skill, an insertion procedure may be associated with a risk of trauma to the cochlea (which may in turn lead to a reduction in residual hearing, pain or discomfort experienced by the patient, etc.), suboptimal electrode lead placement (which may in turn lead to suboptimal cochlear implant system performance, etc.), and/or other undesirable results.

To mitigate such risks, one or more pull wires may be used to facilitate an electrode lead insertion procedure by, for example, being coupled to the electrode lead or a stiffening member associated therewith to allow the electrode lead to be steered and/or otherwise controlled during the insertion procedure. However, it may be difficult or inconvenient for a surgeon to utilize such pull wires when attempting to perform an insertion procedure, particularly when the insertion procedure is performed manually rather than performed using robotic assistance and/or other automatic mechanisms.

U.S. Patent No. 9,211,403 discloses a steerable stylet for inserting an electrode array into a cochlea. The stylet includes a first sensor insertable within a lumen of the electrode array and sensitive to force applied by a lumen wall to the first sensor and a first actuator adapted to move the electrode array in response to the force sensed by the first sensor.

US 5,861,024 relates to a cardiac catheter comprising a pull wire displacement assembly according to the preamble of claim 1.

### BRIEF DESCRIPTION OF THE DRAWINGS

The accompanying drawings illustrate various embodiments and are a part of the specification. The illustrated embodiments are merely examples and do not limit the scope of the disclosure. Throughout the drawings, identical or similar reference numbers designate identical or similar elements.
FIGS. 1A and 1B respectively illustrate an exemplary pull wire displacement assembly prior to and subsequent to a usage of the pull wire displacement assembly to pull an exemplary pull wire according to principles described herein.
FIGS. 2A and 2B respectively illustrate another exemplary pull wire displacement assembly prior to and subsequent to a usage of the other pull wire displacement assembly to pull another exemplary pull wire according to principles described herein.
FIG. 3 illustrates an exemplary cochlear implant system according to principles described herein.
FIG. 4 illustrates a schematic structure of the human cochlea according to principles described herein.
FIG. 5 illustrates an exemplary electrode lead assembly including an electrode lead and a stiffening member for facilitating an insertion of the electrode lead into a cochlea of a patient according to principles described herein.
FIG. 6 illustrates an exemplary insertion procedure of an electrode lead assembly into a cochlea of a patient as facilitated by an exemplary pull wire displacement assembly according to principles described herein.
FIGS. 7A through 7D illustrate exemplary tracks that may be employed by pull wire displacement assemblies described herein according to principles described herein.
FIGS. 8A and 8B illustrate additional exemplary tracks that may be employed by pull wire displacement assemblies described herein according to principles described herein.
FIG. 9 illustrates an exemplary pull wire displacement method according to principles described herein.

### DETAILED DESCRIPTION

The invention relates to a pull wire displacement assembly as defined in claim 1 . The pull wire displacement assemblies described herein may be employed in various applications and contexts where pull wires are used. For example, pull wire displacement assemblies may be employed in any of the examples mentioned above in which pull wires may be coupled to various types of leads, catheters, stylets, and/or other elongate tubes and instruments inserted into the human body as part of various types of surgical operations and/or other medical procedures.

In particular, as will be illustrated and described in more detail below, pull wire displacement assemblies described herein may be employed in electrode lead insertion procedures for cochlear implant systems to facilitate effective procedures in which trauma to the cochlea is minimized, electrode leads are placed optimally, and so forth. In some examples, pull wire displacement assemblies described herein may allow such insertion procedures to be conveniently and effectively performed by surgeons manually. In certain examples, pull wire displacement assemblies described herein may allow surgeons to both advance the lead insertion and manipulate one or more pull wires using only a single hand, thus freeing the other hand for other tasks. While many examples described herein focus on the use of pull wire displacement assemblies in the electrode lead insertion context, however, it will be understood that principles disclosed herein may similarly apply in various other medical and non-medical contexts as may be deemed appropriate by a person of ordinary skill in the relevant art.

In one exemplary implementation, a pull wire displacement assembly may include a housing, a plurality of wire stays each configured to affix to a pull wire that extends along the housing and past a distal end of the housing (e.g., toward any of the leads, catheters, stylets, and/or other tubes or instruments described herein), a slider pin disposed within the housing, and a track disposed within the housing and along which the slider pin is configured to slide. The plurality of wire stays may include a proximal wire stay disposed at a proximal position along the housing and configured to statically affix to the pull wire. The plurality of wire stays may further include a distal wire stay disposed at a distal position along the housing and configured to slidably affix to the pull wire in a manner that allows the pull wire to pass through the distal wire stay along a longitudinal axis of the pull wire while substantially constraining radial displacement of the pull wire at the distal wire stay.

The slider pin may be configured to slide along the track in response to a sliding force applied to the slider pin (e.g., applied by a user of the pull wire displacement assembly by way of a finger slider in contact with the slider pin in certain examples). The track may be configured to direct the slider pin along a trajectory that crosses a straight line between the proximal and distal wire stays. As such, when the slider pin slides along the trajectory in response to an application of the sliding force in a first direction, the slider pin may progressively displace the pull wire away from the straight line to thereby pull a distal portion of the pull wire toward the housing through the distal wire stay. By thus causing the distal portion of the pull wire to be pulled toward the housing, the pull wire displacement assembly may conveniently facilitate any operations that may be associated with manipulating the pull wire in a particular implementation.

Pull wire displacement assembly components such as wire stays, slider pins, tracks, etc., are described herein as being disposed "along" the housing, "within" the housing, and so forth. It will be understood that such terminology may refer to any suitable location with respect to the housing such as a location internal to the housing (e.g., on an internal surface of the housing in examples in which the housing is hollow or includes a cavity within which the components are disposed), a location external to the housing (e.g., on an external surface of the housing in examples in which the housing is a solid body), or any other suitable location with respect to the housing as may serve a particular implementation.

Various benefits may be provided by pull wire displacement assemblies described herein. In particular, as mentioned above, pull wire displacement assemblies described herein may facilitate pull wire manipulation in various types of medical procedures or surgical operations such as electrode lead insertion procedures for cochlear implant system implantation and setup. For example, pull wire displacement assemblies described herein may allow a surgeon to accurately and conveniently perform a manual insertion procedure for a cochlear implant electrode lead using only one hand to both advance the electrode lead into the cochlea and to steer and/or otherwise control (e.g., twist, articulate, etc.) the electrode lead by way of one or more pull wires associated with the electrode lead. In this way, surgeons may avoid costs, setup times, and/or other downsides of insertion procedures performed using non-manual mechanisms and techniques (e.g., robotic, computerized, or other automated mechanisms and techniques), while not compromising patient safety, insertion accuracy, or overall operational effectiveness of the procedure.

As will be made apparent by specific examples described and illustrated below, certain pull wire displacement assembly implementations may provide additional benefits to surgeons performing manual procedures. For example, in certain implementations, pull wire displacement assemblies described herein may provide haptic feedback to help surgeons easily track how far a pull wire has been pulled, may provide support for holding the pull wire in place at a particular position, and so forth. Additionally, the pull wire displacement assemblies and methods described herein may provide a mechanism for making a tradeoff between precision and efficiency in manipulating pull wires. Specifically, by implementing different track gradients (i.e., slopes with respect to the straight line between the wire stays at which the pull wire is affixed), a relationship between a distance across which a sliding force is applied (e.g., a direct distance traveled by a slider pin, a distance traveled by a finger slider in contact with the slider pin, etc.) and a distance by which the pull wire actually moves may be set and optimized in any way as may be desirable. Additionally, a balance between the distance across which the sliding force is applied and an amount of sliding force to be applied to the slider pin may also be optimized by implementing particular track gradients in a similar way.

For example, a track characterized by a relatively mild track gradient (i.e., a mild slope with respect to the straight line between the wire stays) may facilitate a great degree of precision because the sliding force may be applied across a relatively long distance (e.g., a finger slider may move a relatively long distance) to result in a relatively short distance moved by the pull wire. Conversely, a track characterized by a relatively steep track gradient may facilitate a more efficient manipulation of a pull wire because the sliding force may be applied across a relatively short distance to result in a relatively long distance moved by the pull wire. Different implementations of pull wire displacement assemblies may thus be selected by different surgeons according to personal preference, a type of procedure being performed, or the like. Additionally, as will be described in more detail below, different portions of a track having different track gradients may facilitate different amounts of precision and efficiency at different parts of a procedure as may serve a particular implementation.

Various embodiments will now be described in more detail with reference to the figures. The systems and methods described herein may provide one or more of the benefits mentioned above and/or various additional and/or alternative benefits that will be made apparent herein.

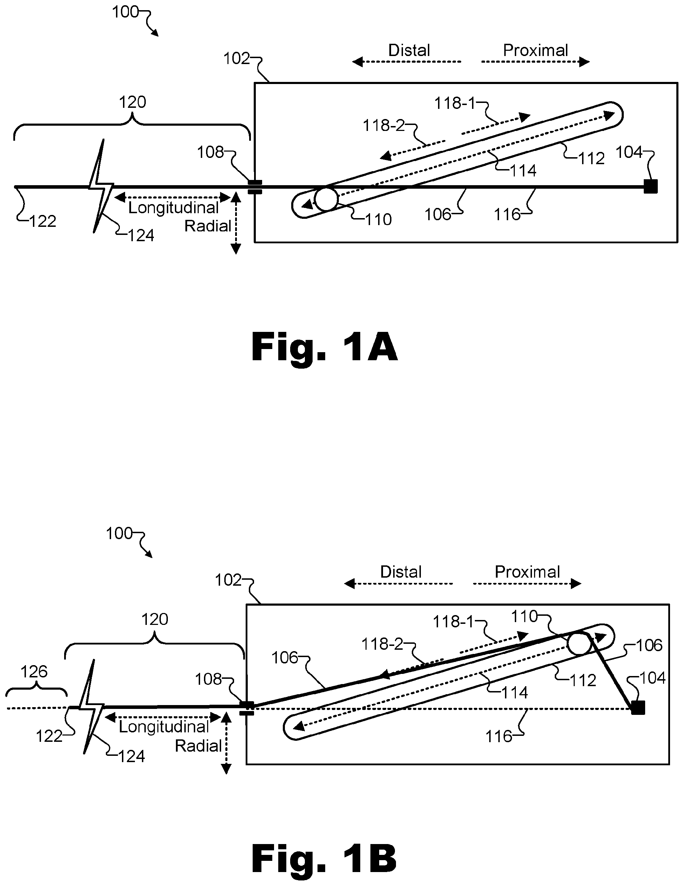

FIGS. 1A and 1B respectively illustrate an exemplary pull wire displacement assembly 100 ("assembly 100") prior to (FIG. 1A) and subsequent to (FIG. 1B) a usage of the pull wire displacement assembly 100 to pull an exemplary pull wire. As shown in both FIGS. 1A and 1B, assembly 100 includes a housing 102 oriented so as to have a distal direction pointing toward an operational site (e.g., a surgery site on a patient) and a proximal direction pointing away from the operational site. In FIG. 1A, housing 102 is illustrated as a box drawn around other components of assembly 100. As described above, each of these components may be associated with housing 102 in any suitable way (e.g., contained within housing 102, disposed on an internal or external surface of housing 102, etc.). A more detailed example of one possible implementation of housing 102 will be illustrated below.

A proximal wire stay 104 is disposed at a proximal position along housing 102, and is configured to statically affix to a pull wire 106 that extends along housing 102 and past a distal end of housing 102. For example, as will be described in more detail below, pull wire 106 may extend past the distal end of housing 102 and through a stiffening member (not shown in FIGS. 1A and 1B) that is associated with an electrode lead and that may be controlled by way of pull wire 106 using assembly 100 during an insertion procedure. Proximal wire stay 104 may be implemented in any way suitable way to statically fix a proximal end of pull wire 106 in place with respect to movements in both longitudinal and radial directions as those directions are indicated in FIGS. 1A and 1B. For example, proximal wire stay 104 may be configured to statically fix pull wire 106 in place by physically restraining it using a clamping mechanism, a friction mechanism, a pinching mechanism, an adhesive mechanism, or any other suitable mechanism.

A distal wire stay 108 is disposed at a distal position along housing 102 and is configured to slidably affix to pull wire 106. Specifically, distal wire stay 108 may fix pull wire 106 in a manner that allows pull wire 106 to pass through distal wire stay 108 along a longitudinal axis of pull wire 106 (i.e., allowing longitudinal movement of pull wire 106) while substantially constraining radial displacement of pull wire 106 at distal wire stay 108. As used herein, displacement of a pull wire may be "substantially constrained" in one or more particular directions (e.g., a radial direction, a longitudinal direction, etc.) when the pull wire is not physically allowed to move significantly in that direction. For example, even if a small degree of displacement is allowed to occur (e.g., as illustrated, for example, in FIG. 1B and in other certain figures below), this displacement may be minimal in comparison to a displacement that would take place if the pull wire were not substantially constrained.

Distal wire stay 108 may perform the function of substantially constraining the radial displacement of pull wire 106 in any suitable way. For example, in contrast to proximal wire stay 104, which substantially constrains both longitudinal and radial movement of pull wire 106, distal wire stay 108 may constrain radial movement of pull wire 106 at distal wire stay 108 even while including a channel or the like through which pull wire 106 is free to run longitudinally such that pull wire 106 may be pulled through the channel. In some examples, distal wire stay 108 may be implemented as a hole or channel within housing 102 through which pull wire 106 may be passed, or may be implemented in any other suitable manner.

As further shown in FIGS. 1A and 1B, a slider pin 110 may be disposed within housing 102. Slider pin 110 may be configured to slide, in response to a sliding force applied to slider pin 110, along a track 112 that is also disposed within the housing. Slider pin 110 may be implemented by any component of any size or shape that is configured to displace pull wire 106 by way of physical contact with pull wire 106 when a contacting portion of slider pin 110 traverses a trajectory 114 provided by track 112. For instance, in the example illustrated in FIGS. 1A and 1B, slider pin 110 may be implemented as a cylindrical pin configured to slide (i.e., move, roll, etc.) along track 112, physically contacting and displacing pull wire 106 from a straight line 116 between wire stays 104 and 108 as it slides.

Accordingly, as shown, track 112 may be configured to direct slider pin 110 along trajectory 114 that crosses straight line 116 between proximal wire stay 104 and distal wire stay 108 such that, when slider pin 110 slides along trajectory 114 in response to an application of the sliding force in a first direction 118-1, slider pin 110 progressively displaces pull wire 106 away from straight line 116. In this way, the movement of slider pin 110 along trajectory 114 may thereby pull a distal portion 120 of pull wire 106 (i.e., a portion of pull wire 106 that is external to housing 102 such as a portion extending through a stiffening member included within an electrode lead assembly or other tube, catheter, or medical device) toward housing 102 through distal wire stay 108.

While pull wire 106 is coincident with straight line 116 in FIG. 1A before and sliding force has been applied, as such a force is applied and slider pin 110 slides up track 112 along trajectory 114 in direction 118-1 to the new position of slider pin 110 in FIG. 1B, FIG. 1B shows that slider pin 110 may radially displace pull wire 106 away from straight line 116. This displacement pulls pull wire 106 partially through distal wire stay 108. Specifically, while a distal tip 122 of pull wire 106 is shown to be at one particular location in FIG. 1A (which may be some distance away from assembly 100 as indicated by a discontinuity symbol 124), distal tip 122 is shown to have been pulled in a proximal direction (i.e., toward housing 102 of assembly 100) by a distance 126 in FIG. 1B. In other words, once slider pin 110 has slid along track 112 in FIG. 1B, part of distal portion 120 of pull wire 106 has been pulled through distal wire stay 108 into housing 102 to thereby pull the remainder of distal portion 120 (including distal tip 122) in the proximal direction toward housing 102.

In some examples, slider pin 110 and track 112 may be configured so that slider pin 110 may only slide along track 112 in a single direction (i.e., direction 118-1) so as to only be support a pulling manipulation of pull wire 106. For example, a ratcheting mechanism may be employed so as to allow slider pin 110 to move only in direction 118-1 up track 112 and not to allow slider pin 110 to slide back down track 112 in a direction 118-2 (e.g., at least until the ratcheting mechanism is released such as when the pull wire has been fully pulled and, for example, an insertion procedure is complete). In other examples, track 112 may be further configured to direct slider pin 110 along trajectory 114 such that, when slider pin 110 slides along the trajectory in response to an application of the sliding force in a second direction opposite to the first direction (i.e., in direction 118-2), slider pin 110 progressively releases the pull wire to allow the pull wire to conform to straight line 116 by permitting distal portion 120 to move away from housing 102 through distal wire stay 108. For instance, by applying a sliding force to slider pin 110 in direction 118-2, a user may release pull wire to a certain extent, thereby allowing distal tip to move back in a distal direction up to an including the initial position of distal tip 122 illustrated in FIG. 1A. This feature may be useful for steering or otherwise controlling a pull wire in examples where, due to a manner in which pull wire 106 is configured (e.g., due to a nature of a device to which distal tip 122 is coupled), pull wire 106 exhibits some resistance to being pulled and is capable of pulling itself back in the distal direction when given leeway to do so.

In FIGS. 1A and 1B, track 112 and trajectory 114 are illustrated as sloping upward from the distal end of housing 102 to the proximal end of housing 102, and slider pin 110 is illustrated as being disposed below pull wire 106 so as to displace pull wire 106 away from straight line 116 in an upward direction when traversing trajectory 114. As shown, these characteristics make direction 118-1 (i.e., the direction in which a sliding force is to be applied to slider pin 110 to pull distal portion 120 toward housing 102) substantially align with the proximal direction. In other words, if, as may be the case in various examples, the sliding force is applied to slider pin 110 by way of a finger slider (e.g., a thumb slider or the like) configured to physically contact slider pin 110 and to slide along an outer surface of housing 102 such as along the top of housing 102, the finger slider may be slid in the proximal direction to effect a pulling manipulation of pull wire 106 in the proximal direction, and may be slid in the distal direction to allow pull wire 106 to move back in the distal direction.

This situation in which a sliding force (e.g., either on slider pin 110 directly or by way of a finger slider in contact with slider pin 110) is applied in a same direction as pull wire 106 is pulled may be intuitive and convenient in certain implementations and/or may be preferred by certain surgeons using assembly 100. However, in other implementations, it may be desirable for a sliding force to be applied in an opposite direction as pull wire 106 is to be pulled (i.e., such that pull wire 106 is pulled in a proximal direction when a sliding force is applied in a distal direction, and pull wire 106 is released to move back in the distal direction when a sliding force is applied in a proximal direction). This alternative configuration may be implemented in a pull wire displacement assembly similar to assembly 100 by having a track that slopes in the same way as track 112, but by configuring slider pin 110 to start above pull wire 106 and to displace pull wire 106 from straight line 116 when slider pin moves along trajectory 114 in direction 118-2 (i.e., displacing pull wire 106 in a downward direction rather than an upward direction). In other examples, this alternative configuration may be implemented by having a track that slopes in an opposite direction as track 112.

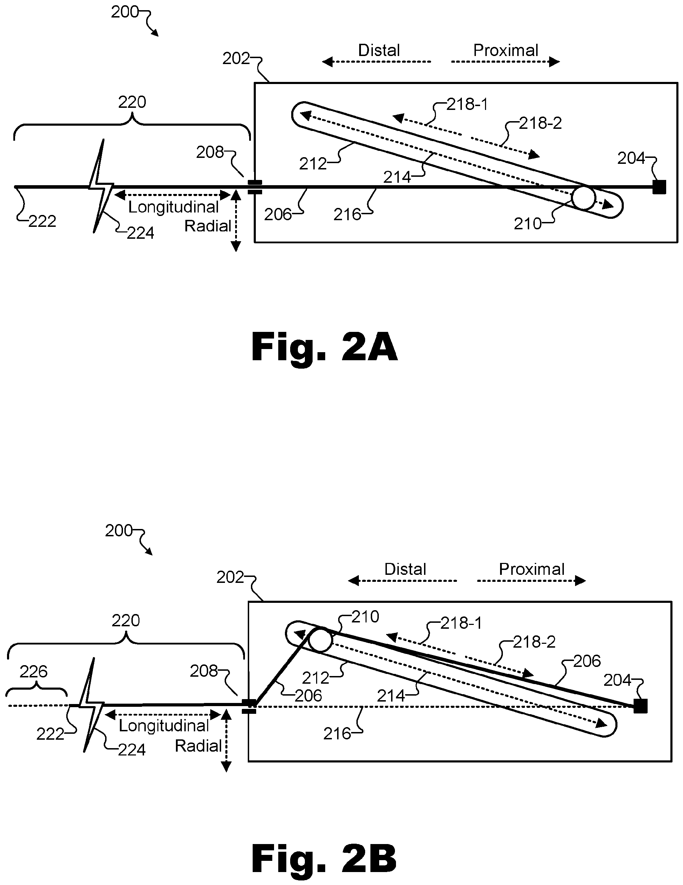

To illustrate, FIGS. 2A and 2B respectively illustrate another exemplary pull wire displacement assembly 200 ("assembly 200") prior to (FIG. 2A) and subsequent to (FIG. 2B) a usage of the other pull wire displacement assembly to pull another exemplary pull wire. As shown, assembly 200 is similar to assembly 100 and may be considered to be an alternative implementation of assembly 100. As shown, assembly 200 includes a housing 202 oriented with distal and proximal directions identical to those illustrated with respect to assembly 100. Assembly 200 further includes a proximal wire stay 204, a pull wire 206, a distal wire stay 208 and a slider pin 210 that each perform identical roles as described for corresponding features in assembly 100.

However, in contrast to assembly 100, a track 212 in assembly 200 that provides a trajectory 214 for pin 210 to slide along is configured to slope upwards from the proximal end of housing 202 to the distal end of housing 202. As such, pull wire 206 is displaced away from a straight line 216 between wire stays 204 and 208 when a sliding force is applied to slider pin 210 in a direction 218-1 that is substantially in the distal direction (i.e., such that a finger slider configured to be in contact with slider pin 210 and to slide along the upper surface of housing 202 would slide in the distal direction to apply the sliding force to slider pin 210 in direction 218-1). When slider pin 210 slides in direction 218-1, a distal portion 220 of pull wire 206 that includes a distal tip 222 (which may be some distance away from housing 202 as indicated by a discontinuity symbol 224) may be pulled in the proximal direction by a distance 226, as illustrated in the change from FIG. 2A to FIG. 2B. Additionally, in some examples, assembly 200 may also allow slider pin 210 to be slid down track 212 in a direction 218-2 to allow pull wire 206 to be pulled back in the distal direction away from housing 202 in a similar way as described above in relation to assembly 100.

As described above, one exemplary context in which pull wire displacement assemblies (e.g., assemblies 100 and/or 200) and/or pull wire displacement methods described herein may be particularly useful is in the context of cochlear implant systems and electrode lead insertion procedures in particular.

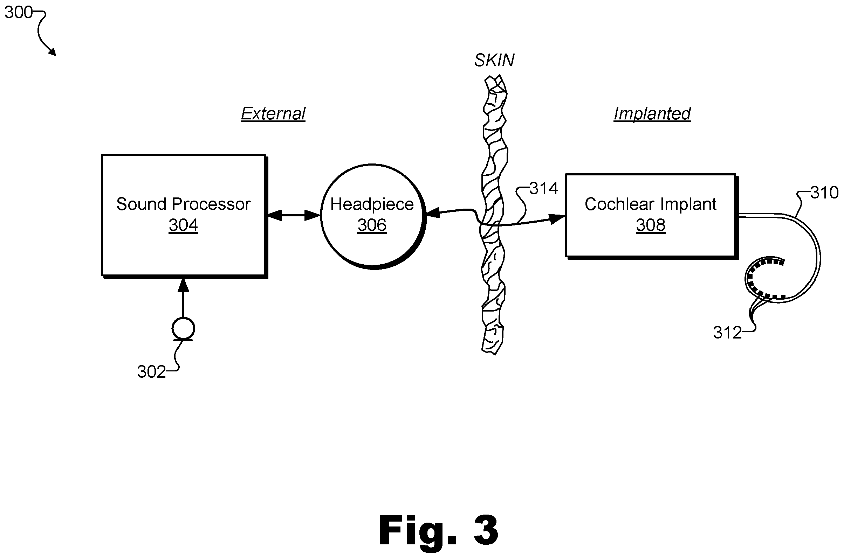

To this end, FIG. 3 illustrates an exemplary cochlear implant system that may be implanted and outfitted to a patient using the pull wire displacement assemblies and methods described herein. As shown, cochlear implant system 300 may include a microphone 302, a sound processor 304, a headpiece 306 having a coil disposed therein, a cochlear implant 308, and an electrode lead 310 including a plurality of electrodes 312. Additional or alternative components may be included within cochlear implant system 300 as may serve a particular implementation.

As shown, cochlear implant system 300 may include various components configured to be located external to a patient including, but not limited to, microphone 302, sound processor 304, and headpiece 306. Cochlear implant system 300 may further include various components configured to be implanted within the patient including, but not limited to, cochlear implant 308 and electrode lead 310.

Microphone 302 may be configured to detect audio signals presented to the patient. Microphone 302 may be implemented in any suitable manner. For example, microphone 302 may include a microphone that is configured to be placed within the concha of the ear near the entrance to the ear canal, such as a T-MIC^{™} microphone from Advanced Bionics. Such a microphone may be held within the concha of the ear near the entrance of the ear canal by a boom or stalk that is attached to an ear hook configured to be selectively attached to sound processor 304. Additionally or alternatively, microphone 302 may be implemented by one or more microphones disposed within headpiece 306, one or more microphones disposed within sound processor 304, one or more beam-forming microphones, and/or any other suitable microphone as may serve a particular implementation.

Sound processor 304 may represent a sound processor having a processing component (e.g., including various computing components such as a processor, memory, communication interfaces, etc.), a battery component, and, in certain implementations, one or more other components such as an earhook component, a cable component (e.g., a cable communicatively coupling sound processor 304 with headpiece 306), and so forth. Sound processor 304 may be configured to process an audio signal (e.g., an acoustic audio signal detected by microphone 302, an electrical audio signal input by way of an auxiliary audio input port or a Clinician's Programming Interface ("CPI") device, etc.) and to direct stimulation representative of the audio signal to be presented to a patient using cochlear implant system 300. For example, the stimulation representative of the audio signal and directed by the sound processor component to be presented to the patient may be electrical stimulation generated by cochlear implant 308 and applied by electrodes 312 on electrode lead 310 implanted within the user.

Sound processor 304 may be configured to direct cochlear implant 308 to generate and apply electrical stimulation (also referred to herein as "stimulation current") representative of an audio signal to the patient. For example, sound processor 304 may direct cochlear implant 308 to apply electrode stimulation to one or more stimulation sites associated with an auditory pathway (e.g., the auditory nerve) of the patient. Exemplary stimulation sites include, but are not limited to, one or more locations within the cochlea, the cochlear nucleus, the inferior colliculus, and/or any other nuclei in the auditory pathway.

Sound processor 304 may process the audio signal in accordance with a selected sound processing strategy or program to generate appropriate stimulation parameters for controlling cochlear implant 308. Sound processor 304 may be housed within any suitable housing. For example, sound processor 304 may be implemented as a behind-the-ear ("BTE") unit, a body worn unit, or the like.

In some examples, sound processor 304 may wirelessly transmit stimulation parameters (e.g., in the form of data words included in a forward telemetry sequence) and/or power to cochlear implant 308 by way of a wireless communication link 314 between headpiece 306 and cochlear implant 308 (e.g., a wireless link between a coil disposed within headpiece 306 and a coil included within or coupled to cochlear implant 308). To this end, headpiece 306 may be communicatively coupled to sound processor 304 and may include an antenna (e.g., a coil and/or one or more wireless communication components) configured to facilitate selective wireless coupling of sound processor 304 to cochlear implant 308. Headpiece 306 may be configured to be affixed to the patient's head and positioned or aligned such that an antenna housed within headpiece 306 is communicatively coupled to a corresponding implantable antenna (which may also be implemented by a coil and/or one or more wireless communication components) included within or otherwise associated with cochlear implant 308. In this manner, stimulation parameters and/or power signals may be wirelessly transferred between sound processor 304 and cochlear implant 308 via wireless communication link 314 transcutaneously.

Cochlear implant 308 may include any type of implantable stimulator that may be used in association with systems described herein. For example, cochlear implant 308 may be implemented by an implantable cochlear stimulator. In some alternative implementations, cochlear implant 308 may include a brainstem implant and/or any other type of cochlear implant that may be implanted within a patient and configured to apply stimulation to one or more stimulation sites located along an auditory pathway of a patient.

Electrode lead 310 may include an array of electrodes 312 disposed on a distal portion of electrode lead 310 and that are configured to be inserted into the cochlea to stimulate the cochlea after the distal portion of electrode lead 310 is inserted into the cochlea. Electrode lead 310 may be inserted into the cochlea in an insertion procedure such as any of the insertion procedures described herein. As such, the insertion of electrode lead 310 may be facilitated by pull wire manipulation using a pull wire displacement assembly such as assembly 100 in any of the ways described herein. Specifically, as electrode lead 310 is being inserted into the cochlea, the pull wire displacement assembly may be used to manipulate pull wires of a stiffening member to which the shape of electrode lead 310 conforms. By so doing, the pull wire displacement assembly may help cause electrode lead 310 to curl and curve so as to properly fit within the spiral shape of the cochlea, as shown.

Electrodes 312 may all be disposed on one side of electrode lead 310 such as an inward side around which electrode lead 310 is configured to curl. For example, electrode lead 310 may be substantially straight and configured to curl around the inward side in conformance with the flexing of a stiffening member inserted into a lumen of the electrode lead or integrated with the electrode lead. In other examples, electrode lead 310 may be pre-curved, or may have a hybrid form that is partly straight and partly pre-curved and that similarly conforms with a flexing of any of the stiffening members described herein. In these ways, electrode lead 310 may be configured to be inserted and arranged in a perimodiolar position, a mid-scalar position, a position near the lateral wall, or any other suitable position or combination of positions along the length of electrode lead 310 as may serve a particular implementation. It will be understood that one or more other electrodes (e.g., a ground electrode, not explicitly shown) may also be disposed on other parts of electrode lead 310 such as on a proximal portion of electrode lead 310 to, for example, provide a current return path for stimulation current generated by electrodes 312.

In some examples, cochlear implant 308 may be configured to generate electrical stimulation representative of an audio signal processed by sound processor 304 (e.g., an audio signal detected by microphone 302) in accordance with one or more stimulation parameters transmitted thereto by sound processor 304. Cochlear implant 308 may be further configured to apply the electrical stimulation to one or more stimulation sites (e.g., one or more intracochlear locations) within the patient by way of electrodes 312 disposed along electrode lead 310. In some examples, cochlear implant 308 may include a plurality of independent current sources each associated with a channel defined by one or more of electrodes 312. In this manner, different stimulation current levels may be applied to multiple stimulation sites simultaneously by way of multiple electrodes 312.

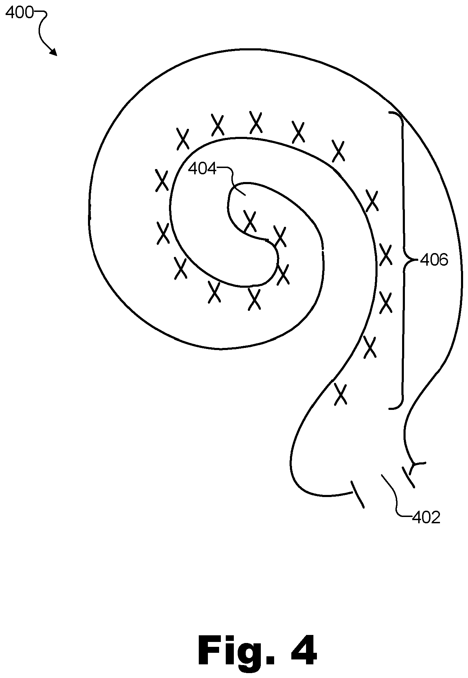

FIG. 4 illustrates a schematic structure of the human cochlea 400 into which electrode lead 310 may be inserted. As shown in FIG. 4, cochlea 400 is in the shape of a spiral beginning at a base 402 and ending at an apex 404. Within cochlea 400 resides auditory nerve tissue 406 (also referred to herein as cochlear tissue), which is denoted by Xs in FIG. 4. The auditory nerve tissue 406 is organized within the cochlea 400 in a tonotopic manner. Relatively low frequencies are encoded at or near apex 404 of cochlea 400 (referred to as an "apical region") while relatively high frequencies are encoded at or near base 402 (referred to as a "basal region"). Hence, electrical stimulation applied by way of electrodes disposed within the apical region (i.e., "apical electrodes") may result in the patient perceiving relatively low frequencies and electrical stimulation applied by way of electrodes disposed within the basal region (i.e., "basal electrodes") may result in the patient perceiving relatively high frequencies. The delineation between the apical and basal electrodes on a particular electrode lead may vary depending on the insertion depth of the electrode lead, the anatomy of the patient's cochlea, and/or any other factor as may serve a particular implementation.

A cochlear implant system with an electrode lead such as electrode lead 310 may operate within a cochlea such as cochlea 400 after the electrode lead has been properly inserted into the cochlea in an effective insertion procedure. Pull wire displacement assemblies and methods described herein may facilitate such effective insertion procedures by allowing surgeons to precisely and conveniently manipulate pull wires configured to control the shape, deflection, articulation, etc., of the electrode lead. For example, when an insertion procedure begins, the electrode lead may be in a substantially linear (i.e., straightened) form that is stiff enough for the electrode lead to be maneuvered into the cochlea without buckling, snagging, and/or encountering other such issues. At the same time, the electrode lead may be flexible enough to easily flex when a flexure force is applied to the electrode lead by way of a pull wire and/or by physical contact with cochlear tissue during the insertion procedure. By so responding to such flexure forces, the electrode lead may flex and/or be steered around the tissue rather than translocating through the tissue or otherwise causing trauma to the tissue.

To this end, a pull wire displacement assembly such as assembly 100 or another implementation thereof (e.g., assembly 200 or any other pull wire displacement assembly described herein) may facilitate manipulation of a pull wire that extends past a housing of the pull wire displacement assembly and along (e.g., through) a stiffening member configured to facilitate insertion of the electrode lead into the cochlea. For example, the stiffening member may include an elongate body that has a first side that is configured to be closer to the electrodes while the body is integrated with the electrode lead, and a second side opposite the first side. The body of the stiffening member may be configured to integrate with a portion of the electrode lead along a length of the electrode lead so as to maintain the portion of the electrode lead in a substantially linear configuration in an absence of a flexure force on the body. The distal tip of the stiffening member may be coupled with the distal portion of the pull wire (e.g., with a distal tip of the pull wire such as distal tip 122 of distal portion 120 in FIGS. 1A and 1B). As such, the pull wire may generate a presence of the flexure force on the body of the stiffening member when a slider pin included within the pull wire displacement assembly progressively displaces the pull wire to thereby pull the distal portion of the pull wire toward the housing. The presence of the flexure force causes the body of the stiffening member to flex inwardly on the first side (i.e., to begin to curl in on the electrodes in a similar fashion as illustrated by electrode lead 310 in FIG. 3), thereby facilitating the electrode lead in conforming to the spiral shape of the cochlea of the patient.

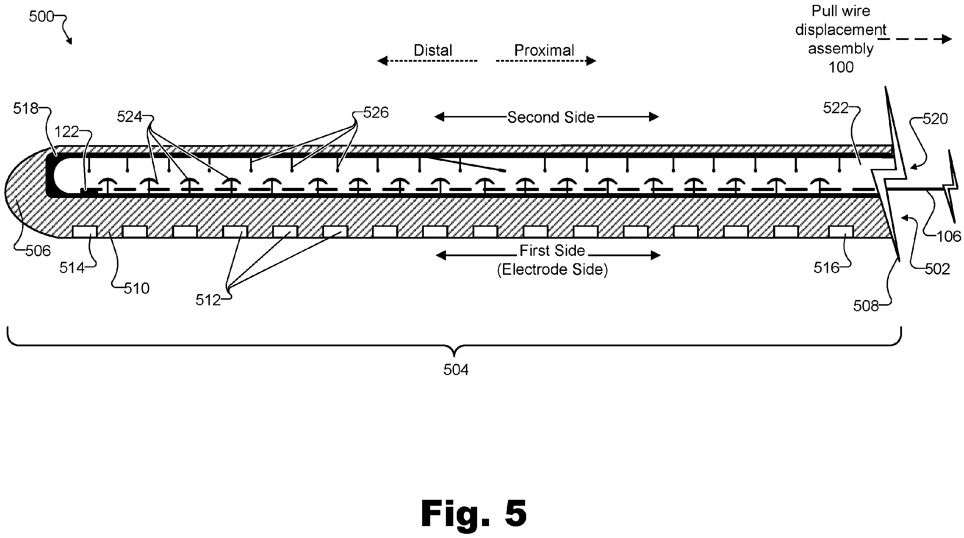

To illustrate an exemplary configuration involving an electrode lead that is steerable by way of a stiffening member coupled with a pull wire, FIG. 5 depicts an exemplary electrode lead assembly 500 including an electrode lead and a stiffening member for facilitating an insertion of the electrode lead into a cochlea of a patient. Specifically, FIG. 5 illustrates electrode lead assembly 500 in a substantially linear configuration such as electrode lead assembly 500 may be in prior to an insertion procedure in which electrode lead assembly 500 is inserted into a cochlea of a patient. As shown, electrode lead assembly 500 includes an electrode lead 502, a distal portion 504 of which is depicted in FIG. 5. While distal portion 504 includes a distal tip 506 of electrode lead 502, it will be understood that a proximal portion 508 of electrode lead 502 is not explicitly illustrated in FIG. 5 but rather is represented by an omission symbol shown at a most proximal point on the portion of electrode lead 502 illustrated. It will be understood that proximal portion 508 of electrode lead 502 may extend past distal portion 504 to couple with a cochlear implant such as cochlear implant 308 beyond the omission symbol.

Electrode lead 502 may include an elongate lead body 510 having a first side and a second side opposite the first side, as labeled in FIG. 5. Along the first side of lead body 510, a plurality of electrodes 512 may be disposed, including a most distal electrode 514 and most proximal electrode 516. While additional electrodes (e.g., such as a ground electrode) may be included on proximal portion 508 (not shown), it will be understood that electrodes 512 disposed along the first side of lead body 510 may not be distributed along the entire length of the entire first side of lead body 510, but, rather, may be limited to distal portion 504, as shown. In a similar way, it will be understood that a stiffening member may only integrate with a certain portion of electrode lead 502, rather than integrating with an entire length of electrode lead 502.

In the example of FIG. 5, electrode lead 502 is shown to further include a lumen 518 into which a stiffening member such as a stylet may be inserted. As such, a stylet 520 may be encapsulated within lumen 518 to facilitate an insertion procedure of electrode lead assembly 500 into a cochlea of a patient. As shown, stylet 520 implements a slotted stiffening member that, as will be described in more detail below, includes an elongate body 522, a plurality of compression slots 524, and a plurality of strain relief slots 526. As will be described below, other types of stylets and/or stiffening members (e.g., that do not include slots) may be employed in other examples as may serve a particular implementation.

In certain examples, stylet 520 may be configured to be temporarily encapsulated in lumen 518 of electrode lead 502 so as to be removable from lumen 518 after a surgical insertion of electrode lead 502 into the cochlea of the patient. Conversely, in other examples, stylet 520 may be configured to be permanently encapsulated in lumen 518 of electrode lead 502 so as to remain encapsulated in lumen 518 after the surgical insertion of electrode lead 502 into the cochlea of the patient.

As shown, body 522 of stylet 520 may have a first side and a second side opposite the first side and may be configured to be encapsulated within lumen 518 of electrode lead 502 so as to maintain electrode lead 502 in a substantially linear configuration in an absence of a flexure force on body 522. The first side of body 522 corresponds to the first side of the electrode lead and, as such, is configured to be closer to electrodes 512 than the second side of body 522 while body 522 is encapsulated within lumen 518 of electrode lead 502.

Body 522 may be constructed of any suitable material with any suitable plasticity limits. For instance, in some implementations, body 522 of stylet 520 may be constructed of a material that will plastically deform as body 522 flexes in the presence of a flexure force. In other words, even after the flexure force is removed, these implementations of stylet 520 may not return to the substantially linear configuration but may at least partially retain the flexed configuration. In other implementations, body 522 of stylet 520 may be constructed of a material that does not plastically deform (e.g., does not reach a limit of plasticity) as a result of an inward flexing of body 522 due to the presence of the flexure force, even when stylet 520 is inwardly flexed to a relatively large angle of deflection (e.g., up to 270°, up to 360°, etc.). In other words, even after such implementations of stylet 520 have been inwardly flexed significantly in the presence of a flexure force, body 522 may return to the substantially linear configuration illustrated in FIG. 5 upon removal of the flexure force causing the inward flexing. Such implementations may be advantageous if it is desirable for stylet 520 to be removed from lumen 518 after electrode lead assembly 500 has been inserted into the cochlea.

In view of these factors, any of various suitable materials may be used to construct stylet 520. For example, a surgical grade stainless steel material (e.g., stainless steel surgical tubing, etc.) or a polymer material (e.g., polyimide tubing, PTFE tubing, etc.) may be used. Additionally, a coating may be applied to the material from which stylet 520 is constructed to reduce friction, protect the material, and so forth. For instance, a PARYLENE coating, PTFE coating, or other suitable coating may be employed.

As shown in FIG. 5, stylet 520 may include compression slots 524 distributed along the first side of body 522, and may include strain relief slots 526 distributed along the second side of body 522. The plurality of compression slots 524 may be configured to compress, in a presence of the flexure force, so as to bias the body to flex in an inward direction (i.e., to curl inward on the first side with the electrodes as illustrated by electrode lead 310 in FIG. 3). At the same time, the plurality of strain relief slots may be configured to expand, in the presence of the flexure force, so as to complement the plurality of compression slots in biasing the body to flex in the inward direction.

Slots 524 and 526 may be formed in any manner as may serve a particular implementation. For example, slots 524 and 526 may be formed in tubing material by way of a micromachining process, by laser cutting, or the like. In other examples, slots 524 and 526 may be formed by way of a molding process or in another suitable manner.

A slotted stiffening member such as stylet 520 may be advantageous for facilitating an insertion procedure involving an electrode lead such as electrode lead 502 because slots 524 and 526 may be configured to bias the stiffening member to only flex in a desired, preconfigured manner (e.g., to only flex in accordance with the spiral-like curvature of a human cochlea). Such slotted stiffening members are described in detail, along with a more detailed explanation of these and other advantages in co-pending PCT Application No. PCT/US17/64035, filed November 30, 2017, and entitled SLOTTED STIFFENING MEMBER FOR FACILITATING AN INSERTION OF AN ELECTRODE LEAD INTO A COCHLEA OF A PATIENT (hereinafter "the co-pending application").

As described in the co-pending application, slotted stiffening members that include asymmetric compression and strain relief slots such as slots 524 and 526 may be biased so as to facilitate only unidirectional flexing. As such, when a single pull wire extends through the slotted stiffening member to be coupled at the distal tip of the slotted stiffening member, the single pull wire may provide sufficient control to steer the electrode lead through the cochlea in an effective insertion procedure. For example, as shown in FIG. 5, pull wire 106 (described above in relation to assembly 100) may extend through stylet 520 to connect to a distal tip of stylet 520 at distal tip 122 of pull wire 106. Because pull wire 106 passes through stylet 520 (i.e., internal to stylet 520), pull wire 106 is illustrated as a dashed line inside stylet 520. However, a solid line beyond proximal portion 508 of electrode lead 502 illustrates that pull wire 106 may continue in the proximal direction to assembly 100, which is not illustrated in FIG. 5. When assembly 100 is used to effect a pulling manipulation on pull wire 106, a flexure force may be applied to stylet 520 by the pull of distal tip 122 where it is coupled to stylet 520. This flexure force may cause compression slots 524 to compress while strain relief slots 526 expand, thereby causing stylet 520 and electrode lead 502 to flex and curl in the inward direction to conform with the cochlear shape.

While FIG. 5 illustrates a unidirectionally-biased stiffening member with asymmetrical compression and strain relief slots, it will be understood that any of the other stiffening members described in the co-pending application or as may be known in the art may similarly benefit from pull wire manipulation operations performed by pull wire displacement assemblies and methods described herein. As another example, bidirectionally-biased stiffening members with symmetrical compression and strain relief slots described in the co-pending application may use two pull wires (one for each of two directions in which the stiffening members are biased to flex) that are both manipulated by a single pull wire displacement assembly using the principles described herein. For instance, a thumb slider may be employed on the pull wire displacement assembly to facilitate pulling a first pull wire that causes the stiffening member to flex in a first direction, while an index finger slider may be employed on an opposite side of the pull wire displacement assembly to facilitate pull a second pull wire that causes the stiffening member to flex in a second, opposite direction.

In still other examples, stiffening members that do not include slots and/or are otherwise not biased to flex in any particular direction may include a plurality of pull wires (e.g., one for each direction up, down, left, and right, or the like) that may be manipulated using one or more pull wire displacement assemblies according to principles described herein.

In certain examples, one or more pull wires may be included within a stiffening member to provide articulation control of the stiffening member (e.g., to prevent unwanted twisting of the stiffening member) in addition to or as an alternative to the pull wire or pull wires configured to facilitate steering control described above. More specifically, a pull wire displacement assembly such as assembly 100 may be configured to facilitate pulling a distal portion of an additional pull wire toward housing 102, and the distal portion of the additional pull wire may be further coupled with the distal tip of the stiffening member in a manner that allows the additional pull wire to generate a presence of an articulation force on the body of the stiffening member when the additional pull wire is pulled toward housing 102. The presence of the articulation force may cause the body of the stiffening member to twist along a longitudinal axis of the stiffening member to thereby further facilitate the electrode lead in conforming to the shape of the cochlea. For example, if the electrode lead and the stiffening member encapsulated therein begin to twist along the longitudinal axis, assembly 100 may be used to pull the additional pull wire to generate the articulation force and to thereby correct the unwanted twisting.

When used together to facilitate an electrode lead insertion procedure for a cochlear implant patient, a combination of a stiffening member, a pull wire coupled to the stiffening member, and a pull wire displacement assembly configured to facilitate manipulation of the pull wire may be referred herein as an electrode lead insertion assembly. For instance, an exemplary electrode lead insertion assembly may include a stiffening member configured to facilitate an insertion of an electrode lead into a cochlea of a patient. The stiffening member may include an elongate body that has a first side (configured to be closer to the electrodes while the body is integrated with the electrode lead) and a second side opposite the first side and that is configured to integrate with a portion of the electrode lead along a length of the electrode lead so as to maintain the portion of the electrode lead in a substantially linear configuration in an absence of a flexure force on the body. In some examples, the stiffening member may be implemented as a slotted stiffening member (e.g., stylet 520) or any other stiffening member described in the co-pending application.

The exemplary electrode lead insertion assembly may further include a pull wire that extends along the stiffening member and is coupled, at a distal portion of the pull wire, with a distal tip of the stiffening member. Additionally, the exemplary electrode lead insertion assembly may include a pull wire displacement assembly similar or identical to assembly 100 or assembly 200 described above. For example, the pull wire displacement assembly may include a housing, a proximal wire stay disposed at a proximal position along the housing and configured to statically affix to the pull wire, a distal wire stay disposed at a distal position along the housing and configured to slidably affix to the pull wire in a manner that allows the pull wire to pass through the distal wire stay along a longitudinal axis of the pull wire while substantially constraining radial displacement of the pull wire at the distal wire stay, and a slider pin disposed within the housing. As mentioned above, but not explicitly illustrated for assemblies 100 or 200, the pull wire displacement assembly may further include a finger slider (e.g., a thumb slider or a finger slider for any other finger as may serve a particular implementation) that is configured to slide along the housing in response to a force applied to the finger slider by a user of the pull wire displacement assembly (e.g., by a surgeon).

Additionally, as with assemblies 100 and 200, the exemplary pull wire displacement assembly may include a track disposed within the housing and along which the slider pin is configured to slide in response to a transfer of the force applied to the finger slider by the user to the slider pin by way of physical contact between the finger slider and the slider pin. The track may be configured to direct the slider pin along a trajectory that crosses a straight line between the proximal and distal wire stays such that, when the user applies the force to the finger slider to cause the slider pin to slide along the trajectory in a first direction, the slider pin progressively displaces the pull wire away from the straight line. In this way, the exemplary pull wire displacement assembly may thereby pull a distal portion of the pull wire toward the housing through the distal wire stay, generate a presence of the flexure force on the body of the stiffening member, and cause the body of the stiffening member to flex inwardly on the first side to thereby facilitate the electrode lead in conforming to a shape of the cochlea of the patient.

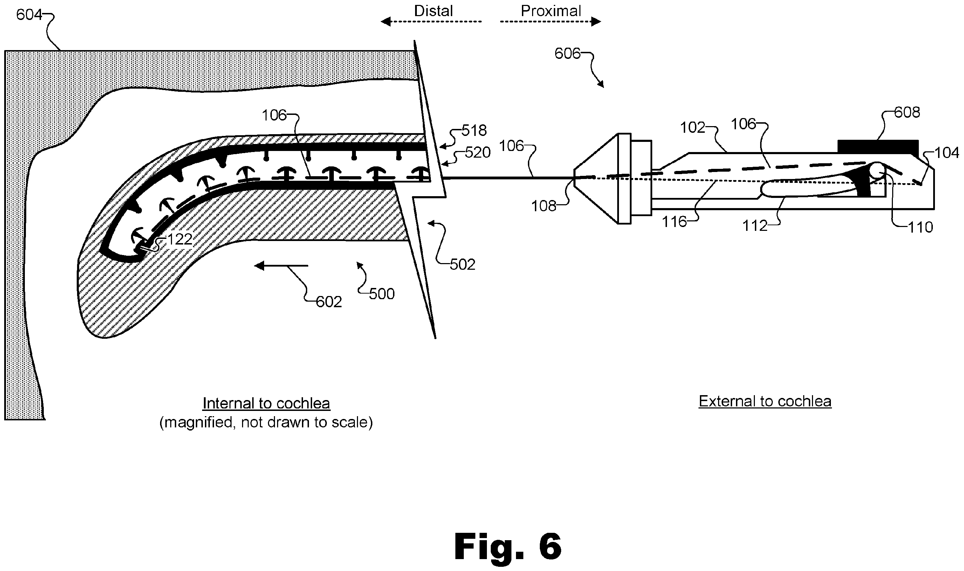

To illustrate, FIG. 6 shows an exemplary insertion procedure 602 of electrode lead assembly 500 into a cochlea 604 of a patient as facilitated by an implementation 606 of assembly 100 (hereinafter "pull wire displacement assembly 606" or "assembly 606"). As shown, an implementation of electrode lead assembly 500 of FIG. 5 includes electrode lead 502 and the slotted stiffening member of stylet 520. Electrode lead assembly 500 is depicted to be flexing under a flexure force present as a result of tension applied to pull wire 106, which is coupled with a distal tip of stylet 520 during insertion procedure 602. The implementation of electrode lead assembly 500 illustrated in FIG. 6 includes electrode lead 502 with lumen 518 in which stylet 520 is encapsulated. It will be understood, however, that various aspects of electrode lead assembly 500 and/or components thereof (e.g., the electrodes on electrode lead 502) that are not specifically shown or labeled in FIG. 6 for clarity may be present and may function as described above in relation to electrode lead assembly 500.

As shown, electrode lead assembly 500 is being inserted into cochlea 604 by way of insertion procedure 602. At a moment during insertion procedure 602 depicted in FIG. 6, electrode lead assembly 500 has begun to flex even though it has not made physical contact with tissue of cochlea 604. This is because electrode lead assembly 500 is flexing under the influence of a flexure force generated by tension on pull wire 106, which is coupled (e.g., welded, etc.) to a distal tip of stylet 520 and is being pulled by assembly 606.

As shown, when the tension is applied to pull wire 106 (e.g., by a surgeon performing insertion procedure 602 using assembly 606 to facilitate manipulating pull wire 106), some of the compression slots on stylet 520 have compressed, some of the strain relief slots have expanded, and the body of stylet 520 has flexed in accordance with the inward flex biasing implemented by the slots. Because pull wire 106 may guide and control stylet 520 to flex electrode lead assembly 500 in this way, insertion procedure 602 may be performed by a surgeon by hand, without robotic or computerized assistance, and using minimal tools including, in some examples, only pull wire displacement assembly 606.

As the surgeon inserts electrode lead assembly 500 into cochlea 604, stylet 520 may be steered using pull wire 106 to flex electrode lead assembly 500 to conform to the curvature of cochlea 604 without causing cochlear trauma such as a translocation. Indeed, in certain examples, the surgeon may steer electrode lead assembly 500 into cochlea 604 with little or no contact at all between the distal tip of electrode lead 502 and the tissue of cochlea 604. As will be described in more detail below, active steering of electrode lead assembly 500 into cochlea 604 may be based on pre-planning or any suitable type of intraoperative monitoring or feedback.

In some examples, a surgeon may perform insertion procedure 602, including actively steering electrode lead assembly 500, using only one hand. While not explicitly shown, stylet 520 may connect to or at least be in physical contact with a distal end of housing 102 (i.e., at distal wire stay 108 where pull wire 106 leaves assembly 606). Thus, for example, by moving assembly 606 in the distal direction, the surgeon may advance electrode lead assembly 500 deeper into cochlea 604 while simultaneously using a finger slider 608 to flex and steer electrode lead assembly 500 by pulling pull wire 106 in the proximal direction. For example, as illustrated, finger slider 608 may be configured to slide along housing 102 in response to a force applied to the finger slider by the surgeon, and to apply the sliding force to slider pin 110 by transferring the force applied to finger slider 608 to slider pin 110 by way of physical contact with slider pin 110. Accordingly, by applying a sliding force to move finger slider 608 in the proximal direction, the surgeon may force slider pin 110 to slide along the trajectory provided by track 112 to displace, and thereby pull through distal wire stay 108, pull wire 106 (illustrated by a dashed line within housing 102 of assembly 606). In some examples, once insertion procedure 602 is complete, stylet 520 may be withdrawn from electrode lead assembly 500 and detached from assembly 606 along with detaching pull wire 106 from wire stays 104 and 108. In other examples, stylet 520 may be configured to remain encapsulated within electrode lead 502 after insertion procedure 602 is complete. Thus, stylet 520 and/or pull wire 106 may be detached from assembly 606 at the end of insertion procedure 602 and may remain implanted within the patient.

In other examples, an implementation of assembly 606 may further include an additional finger slider configured to advance electrode lead assembly 500 deeper into cochlea 604 without moving assembly 606 itself. For instance, a surgeon may use one finger (e.g., a thumb) to steer electrode lead assembly 500 by pulling pull wire 106 while simultaneously or alternately using another finger (e.g., an index finger) to advance electrode lead assembly 500 forward into cochlea 604 (i.e., in the distal direction). In such examples, assembly 600 may be mounted or held so as to remain stationary with respect to the patient while the surgeon is able to perform insertion procedure 602 with one hand using the stationary assembly 606.

As mentioned above, it may be possible to set and/or optimize a relationship between a distance across which a sliding force is applied (e.g., a distance traveled by finger slider 608 and/or slider pin 110) and a distance by which the pull wire actually moves (and, accordingly, a deflection angle by which electrode assembly 500 is flexed) by implementing different track gradients with respect to a straight line between wire stays 104 and 108. Similarly, as further mentioned above, differing track gradients may help manage or optimize a balance between the distance across which the sliding force is applied and an amount of sliding force to be applied to slider pin 110 (i.e., by way of force applied to finger slider 608).

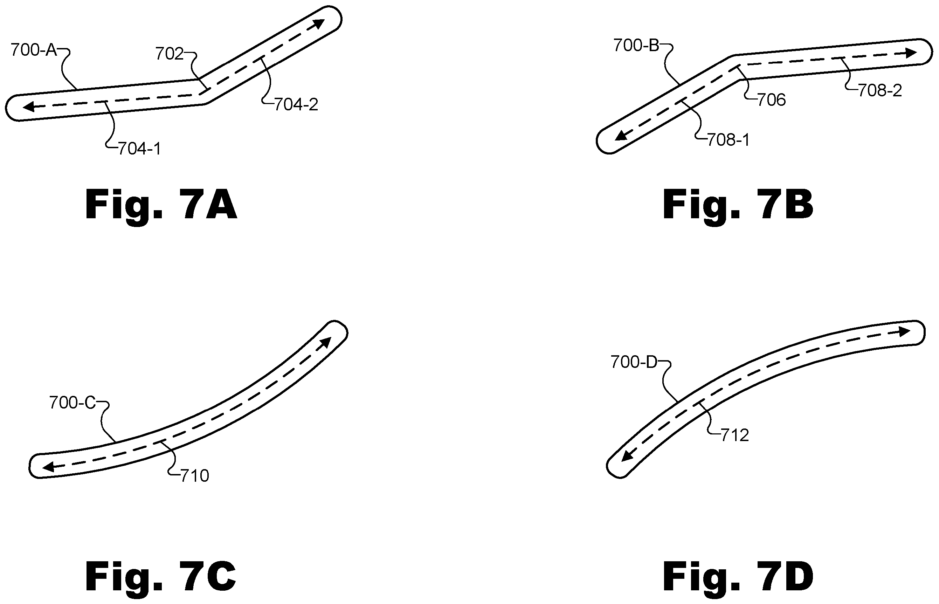

To illustrate, FIGS. 7A through 7D show exemplary tracks 700 (i.e., track 700-A in FIG. 7A, track 700-B in FIG. 7B, track 700-C in FIG. 7C, and track 700-D in FIG. 7D) that may be employed by implementations of pull wire displacement assemblies described herein. For each track 700 illustrated in FIGS. 7A through 7D, it may be assumed that a sliding pin sliding along the track begins at the bottom at a distal side of the track (i.e., on the left-hand side) and travels to toward the top at a proximal side of the track (i.e., on the right-hand side), similar to track 112 shown in assembly 100 in FIGS. 1A and 1B.

As shown, the trajectories along which certain tracks 700 are configured to direct slider pins may include a plurality of linear segments each having different slopes. For example, as illustrated in FIG. 7A, a trajectory 702 along which track 700-A is configured to direct a slider pin may include a first linear segment 704-1 having a first slope (i.e., a relatively mild slope allowing for more precision because a finger slider moving horizontally has to slide a greater distance to displace the pull wire by a particular amount), and a second linear segment 704-2 having a second, different slope (i.e., a relatively steep slope allowing for more efficiency because the finger slider can displace the pull wire by the particular amount by sliding a lesser distance). Track 700-A may thus be well-suited for an insertion procedure requiring a greater degree of precision in an early part of the procedure than in the later part of the procedure, for example.

Similarly, as another example illustrated in FIG. 7B, a trajectory 706 along which track 700-B is configured to direct a slider pin may include a first linear segment 708-1 having a first slope (i.e., a relatively steep slope allowing for more efficiency), and a second linear segment 708-2 having a second, different slope (i.e., a relatively mild slope that prioritizes accuracy over efficiency). Track 700-B may thus be well-suited for an insertion procedure requiring a greater degree of precision in a later part of the procedure than in the earlier part of the procedure, for example. Additionally or alternatively, track 700-B and trajectory 706 may be well-suited to an application where a pull wire becomes harder to pull as the pull wire becomes tighter, and where it is desirable for the amount of force being applied to a finger slider to remain consistent.

Just as trajectories may include more than one linear segment having different slopes, in the same or other examples, the trajectories along which certain tracks 700 are configured to direct the slider pins may include non-linear curves. For example, as illustrated in FIG. 7C, a trajectory 710 along which track 700-C is configured to direct a slider pin may be curved so as to get gradually steeper as a slider pin moves from the distal end to the proximal end. Such a trajectory may be useful, for example, in similar examples as described above for trajectory 702 of FIG. 7A, but where it may be desirable to have a smooth transition from the mild gradient to the steep gradient. In like manner, as illustrated in FIG. 7D, a trajectory 712 along which track 700-D is configured to direct a slider pin may be curved so as to get gradually less and less steep as the slider pin moves from the distal end to the proximal end. Such a trajectory may be useful, for example, in similar examples as described above for trajectory 706 of FIG. 7B, but where it may be desirable to have a smooth transition from the steep gradient to the mild gradient.

Various other shapes of tracks and trajectories besides those illustrated in FIGS. 7A through 7D or in other figures herein may also be useful in certain circumstances. For example, certain tracks and trajectories that may be used in accordance with the systems and methods described herein may include both linear and curved, non-linear portions as may serve a particular implementation.

In each example illustrated above, track surfaces have been shown to be relatively smooth so to allow slider pins to move uniformly across each point of the track surface. Such track surfaces may be desirable in certain examples or may be preferred by certain users (e.g., surgeons) due to the fact that smooth surfaces allow pull wires to be pulled and/or released in a smooth, consistent manner. However, in other examples or in accordance with other user preferences, it may be desirable for pull wires to be manipulated in discrete intervals. For example, a certain length of pull wire may be pulled for each interval of distance that an electrode lead assembly is advanced (e.g., to cause a 10° deflection angle in the flexed electrode lead assembly, for instance, for each 4 mm of lead advancement into the cochlea, etc.). Additionally or alternatively, it may be desirable for certain users to receive haptic feedback indicative of how far a pull wire has been pulled, or, equivalently, to a current deflection angle of a flexed electrode lead assembly. Moreover, it may be desirable in the same or other examples for the slider pin to be retained, to at least some degree, in certain discrete positions. In this way, for example, slight movements of the finger slider (e.g., caused by tremoring hands of the user or the like) may be less likely to result in unwanted tremoring of the electrode lead assembly and/or other unintended steering operations.

To this end, certain tracks providing trajectories along which slider pins are configured to slide may include a plurality of discrete pin advancement features each configured to haptically indicate, to a user of the pull wire displacement assembly applying the sliding force to the slider pin, when the slider pin has advanced along the trajectory from one pin advancement feature to another pin advancement feature in the plurality of discrete pin advancement features.

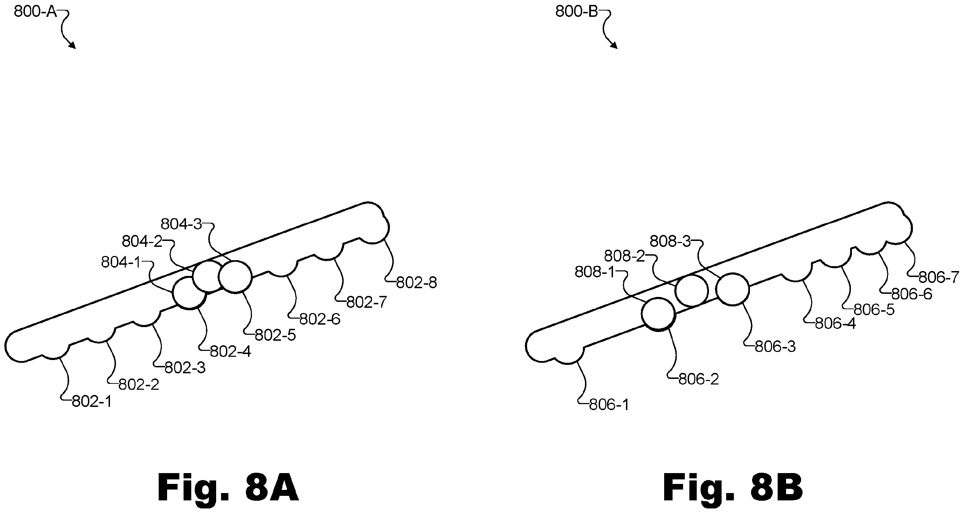

To illustrate, FIGS. 8A and 8B illustrate additional exemplary tracks 800 (i.e., track 800-A in FIG. 8A and track 800-B in FIG. 8B) that may be employed by pull wire displacement assemblies described herein and that each implement discrete pin advancement features to provide benefits described above. Specifically, a plurality of discrete pin advancement features 802 (i.e., features 802-1 through 802-8) are disposed on track 800-A at equal intervals. As shown, each feature 802 may be implemented as a slight indentation within which a slider pin may rest. To illustrate, a slider pin is illustrated in three different positions 804 labeled as position 804-1 (where the slider pin is resting at feature 802-4), position 804-2 (where the slider pin is moving between features 802-4 and 802-5), and position 804-3 (where the slider pin is resting at feature 802-5).

As the slider pin is moved between positions 804 and moved along track 800 to rest at other features 802, haptic feedback may be provided to a user providing the sliding force that causes the slider pin to move. For example, as the slider pin drops into each feature, the user may feel that a relatively small amount of force is needed to keep the slider pin in position, but that a relatively large amount of force is needed to move the slider pin to a different discrete position (e.g., to a different feature 802). In some examples, features 802 may be configured to retain the slider pin in place when the user ceases applying the sliding force to the slider pin (i.e., such that no force is needed to keep the slider pin in position, only to move it to a different feature 802). The amount of force required to hold the slider pin in position and/or to move it out of position toward a different feature 802 may arise from how prominent (i.e., how deep or pronounced) each feature 802 is made to be. As such, each feature 802 does not need to have the same prominence as every other feature 802, but, rather, certain features 802 may be less pronounced (e.g., shallower) while other features 802 may be more pronounced (e.g., deeper) as may serve a particular implementation.

In FIG. 8A, all the features 802 are shown to be distributed equally along track 800. Thus, for example, haptic feedback provided by each feature 802 may represent a certain distance that a pull wire is pulled, a certain degree of deflection with which a stiffening member is flexed, or the like. For instance, if a pull wire, when fully pulled, is configured to flex a particular stiffening member by 80°, each feature 802 may be associated with 10° of deflection and a user of a pull wire displacement assembly implementing track 800 may receive haptic feedback and track the current degree of deflection by counting how many pin advancement features have been passed.

FIG. 8B is similar to FIG. 8A. For example, track 800-B is similar to track 800-A in that a plurality of discrete pin advancement features 806 (i.e., features 806-1 through 806-7) similar to features 802 are disposed. However, whereas features 802 in FIG. 8A are disposed at equal intervals on track 800-A, features 806 on track 800-B are disposed at unequal intervals. As in FIG. 8A, FIG. 8B illustrates a slider pin in three different positions 808 labeled as position 808-1 (where the slider pin is resting at feature 806-2), position 808-2 (where the slider pin is moving between features 806-2 and 806-3), and position 808-3 (where the slider pin is resting at feature 806-3). The same haptic feedback and pin retention benefits described above in relation to features 802 may be similarly provided by features 806 on track 800-B, and features 806 may similarly be characterized by difference prominence in certain implementations as described above.

In FIG. 8B, all the features 806 are shown to be distributed unequally along track 800-B, getting further apart toward the distal end of track 800-B on the left-hand side and getting closer together toward the proximal end of track 800-B on the right-hand side. Thus, rather than representing a certain distance that a pull wire is pulled or a certain degree of deflection with which a stiffening member is flexed, as described above for track 800-A in FIG. 8A, haptic feedback received from the slider pin resting in each of features 806 may represent a particular predetermined milestone associated with an insertion procedure or other such event or indication. This may be useful, for example, if there are certain parts of an insertion procedure (e.g., certain curves of the cochlea) that are associated with certain steering operations. For example, each feature 806 may correspond to a particular turn in the cochlea or the like, and may be "selected" (i.e., by moving the pin to the particular feature 806) as the surgeon advances the electrode lead assembly to each particular turn in the cochlea.

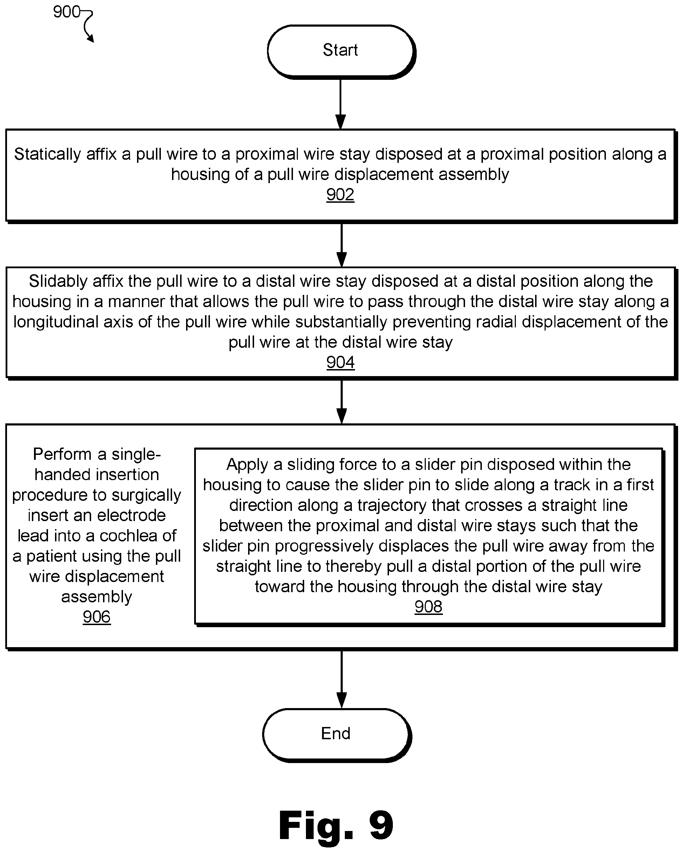

FIG. 9 illustrates an exemplary pull wire displacement method 900 according to principles described herein. While FIG. 9 illustrates exemplary operations according to one embodiment, other embodiments may omit, add to, reorder, and/or modify any of the operations shown in FIG. 9. One or more of the operations shown in FIG. 9 may be performed by a surgeon, by a manufacturer or distributor of a pull wire displacement assembly, or by any other user preparing for, performing, or otherwise facilitating an electrode lead insertion procedure on a cochlear implant patient using a pull wire displacement assembly (e.g., pull wire displacement assembly 100), any components included therein, and/or any implementation thereof.

In operation 902, a surgeon performing an electrode lead insertion procedure on a cochlear implant patient may statically affix a pull wire to a proximal wire stay disposed at a proximal position along a housing of a pull wire displacement assembly. For example, the pull wire may extend along the housing, past a distal end of the housing, and through a stiffening member disposed within a lumen of an electrode lead. The pull wire may then be coupled to a distal tip of the stiffening member. Operation 902 may be performed in any suitable manner in accordance with how the proximal wire stay is implemented, which may be any of the ways described herein.

In operation 904, the surgeon may slidably affix the pull wire to a distal wire stay disposed at a distal position along the housing. For example, the surgeon may thread the pull wire through the distal wire stay or otherwise affix the pull wire in any suitable manner that allows the pull wire to pass through the distal wire stay along a longitudinal axis of the pull wire while substantially constraining radial displacement of the pull wire at the distal wire stay. Operation 904 may be performed in any suitable manner in accordance with how the distal wire stay is implemented, which may be any of the ways described herein.

In operation 906, the surgeon may perform a single-handed insertion procedure to surgically insert the electrode lead into a cochlea of a patient using the pull wire displacement assembly. Operation 906 may be performed in any of the ways described herein. For example, operation 906 may be performed by performing one or more operations including operation 908.

In operation 908, the surgeon may apply a sliding force to a slider pin disposed within the housing to cause the slider pin to slide along a track in a first direction. In some examples, the track may be disposed within the housing and may be configured to direct the slider pin along a trajectory that crosses a straight line between the proximal and distal wire stays such that the slider pin progressively displaces the pull wire away from the straight line to thereby pull a distal portion of the pull wire toward the housing through the distal wire stay. Operation 908 may be performed in any of the ways described herein.

As mentioned above, in certain examples, medical procedures involving pull wires and that are facilitated by pull wire displacement assemblies described herein may be performed based on pre-planning and/or live feedback received during the procedures. For instance, an insertion procedure that includes a performance of method 900 to make use of a pull wire displacement assembly may be performed by the surgeon by applying the sliding force to the slider pin based on pre-operative imaging of the cochlea of the patient or other preplanning data. For example, when a surgeon knows that the distal tip of an electrode lead assembly is approaching a particular curve or turn within the cochlea based on the pre-operative imaging, the surgeon may move the thumb slider by a certain amount (e.g., move it so as to advance the slider pin to the next feature 802 or 806) to properly flex the electrode lead assembly. In the same or other examples, real-time intraoperative imaging may similarly be used such that the surgeon may monitor the position of the distal tip in real time during the insertion procedure.

Additionally or alternatively, the insertion procedure including the performance of method 900 may be performed by applying the sliding force to the slider pin based on real-time intraoperative feedback received from a cochlear implant system in which the electrode lead is included. For example, real-time electrocochleographic sensing may be used to obtain evoked potentials in response to acoustic stimulation provided to the patient, real-time impedance sensing may be used to determine the impedance of cochlear tissue at a particular location at which the distal tip of an electrode lead assembly is located, or other real-time feedback based on other sensing techniques (e.g., pressure sensing, optical sensing, etc.) may be employed. As these or other techniques provide real-time feedback (e.g., feedback indicative of where the advancing electrode lead assembly is located within the cochlea, a likelihood that the advancing electrode lead assembly is causing trauma to cochlear tissue, etc.), the surgeon may use the real-time feedback to make decisions about further advancement of the electrode lead assembly, about how and when to use the pull wire displacement assembly to manipulate the one or more pull wires controlling the flexing of the advancing electrode lead assembly, and so forth.

In the preceding description, various exemplary embodiments have been described with reference to the accompanying drawings. It will, however, be evident that various modifications and changes may be made thereto, and additional embodiments may be implemented, without departing from the scope of the invention as set forth in the claims that follow. For example, certain features of one embodiment described herein may be combined with or substituted for features of another embodiment described herein. The description and drawings are accordingly to be regarded in an illustrative rather than a restrictive sense.

## Claims

1. A pull wire displacement assembly comprising:
a housing (102, 202);
a proximal wire stay (104, 204) disposed at a proximal position along the housing and configured to statically affix to a pull wire (106, 206) that extends along the housing and past a distal end of the housing;
a distal wire stay (108, 208) disposed at a distal position along the housing and configured to slidably affix to the pull wire in a manner that allows the pull wire to pass through the distal wire stay along a longitudinal axis of the pull wire while substantially constraining radial displacement of the pull wire at the distal wire stay;
a slider pin (110, 210) disposed within the housing; and
a track (112, 212, 700, 800) disposed within the housing and along which the slider pin is configured to slide in response to a sliding force applied to the slider pin, the track configured to direct the slider pin along a trajectory (114, 214, 702, 706, 710, 712),
**characterized in that** the trajectory crosses a straight line (116, 216) between the proximal and distal wire stays such that, when the slider pin slides along the trajectory in response to an application of the sliding force in a first direction, the slider pin progressively displaces the pull wire away from the straight line to thereby pull a distal portion (120, 220) of the pull wire toward the housing through the distal wire stay,

2. The pull wire displacement assembly of claim 1, wherein:
past the distal end of the housing (102, 202), the pull wire (106, 206) extends along a stiffening member (520) configured to facilitate an insertion of an electrode lead (310, 502) into a cochlea of a patient, the stiffening member including an elongate body (522) that has a first side and a second side opposite the first side and that is configured to integrate with a portion of the electrode lead along a length of the electrode lead so as to maintain the portion of the electrode lead in a substantially linear configuration in an absence of a flexure force on the body, the first side configured to be closer to the electrodes (312, 512) than the second side while the body is integrated with the electrode lead;
the distal portion (120, 220) of the pull wire is coupled with a distal tip of the stiffening member;
the pull wire generates a presence of the flexure force on the body of the stiffening member when the slider pin progressively displaces the pull wire to thereby pull the distal portion of the pull wire toward the housing; and
the presence of the flexure force causes the body of the stiffening member to flex inwardly on the first side to thereby facilitate the electrode lead in conforming to a shape of the cochlea (400) of the patient.

3. The pull wire displacement assembly of claim 2, wherein the stiffening member (520) further includes:
a plurality of compression slots (524) distributed along the first side of the body, the plurality of compression slots configured to compress, in the presence of the flexure force, so as to bias the body (522) to flex inwardly on the first side; and
a plurality of strain relief slots (526) distributed along the second side of the body, the plurality of strain relief slots configured to expand, in the presence of the flexure force, so as to complement the plurality of compression slots in biasing the body to flex inwardly on the first side.

4. The pull wire displacement assembly of claim 2, wherein:
the pull wire displacement assembly (100, 200) is configured to facilitate pulling a distal portion of an additional pull wire toward the housing (102, 202);
the distal portion of the additional pull wire is further coupled with the distal tip of the stiffening member (520) in a manner that allows the additional pull wire to generate a presence of an articulation force on the body (522) of the stiffening member when the additional pull wire is pulled toward the housing; and
the presence of the articulation force causes the body of the stiffening member to twist along a longitudinal axis of the stiffening member to thereby further facilitate the electrode lead (310, 502) in conforming to the shape of the cochlea (400).

5. The pull wire displacement assembly of claim 1, wherein the track (112, 212, 700, 800) includes a plurality of discrete pin advancement features (802, 806) each configured tc haptically indicate, to a user of the pull wire displacement assembly applying the sliding force to the slider pin (110, 210), when the slider pin has advanced along the trajector (114, 214, 702, 706, 710, 712) from one pin advancement feature to another pin advancement feature in the plurality of discrete pin advancement features.

6. The pull wire displacement assembly of claim 5, wherein the discrete pin advancement features (802, 806) are disposed along the track (112, 212, 700, 800) at unequal intervals.

7. The pull wire displacement assembly of claim 5, wherein the discrete pin advancement features (802, 806) are each further configured to retain the slider pin (110, 210) in place when the user ceases applying the sliding force to the slider pin.

8. The pull wire displacement assembly of claim 1, wherein the trajectory (702, 706) along which the track (700) is configured to direct the slider pin (110, 210) includes a plurality of linear segments (704-1, 704-2, 708-1, 708-2) each having different slopes.

9. The pull wire displacement assembly of claim 1, wherein the trajectory (710, 712) along which the track (700) is configured to direct the slider pin (110, 210) includes a non-linear curve.

10. The pull wire displacement assembly of claim 1, further comprising a finger slider (608) configured to:
slide along the housing (102, 202) in response to a force applied to the finger slider by a user of the pull wire displacement assembly (100, 200); and
apply the sliding force to the slider pin (110, 210) by transferring the force applied to the finger slider to the slider pin by way of physical contact with the slider pin.

11. The pull wire displacement assembly of claim 1, wherein the track (112, 212, 700, 800) is further configured to direct the slider pin (110, 210) along the trajectory such that, when the slider pin slides along the trajectory (114, 214, 702, 706, 710, 712) in response to an application of the sliding force in a second direction opposite to the first direction, the slider pin progressively allows the pull wire (106, 206) to conform to the straight line (116, 216) to thereby permit the distal portion (120, 220) of the pull wire to move away from the housing through the distal wire stay (108, 208) .

12. An electrode lead insertion assembly comprising:
a stiffening member (520) configured to facilitate an insertion of an electrode lead (310, 502) into a cochlea (400) of a patient, the stiffening member including an elongate body (522) that has a first side and a second side opposite the first side and that is configured to integrate with a portion of the electrode lead along a length of the electrode lead so as to maintain the portion of the electrode lead in a substantially linear configuration in an absence of a flexure force on the body, the first side configured to be closer to the electrodes (312, 512) than the second side while the body is integrated with the electrode lead;
the pull wire displacement assembly (100, 200) of claim 10; and
the pull wire (106, 206), wherein the pull wire extends along the stiffening member and is coupled, at a distal portion (120, 220) of the pull wire, with a distal tip of the stiffening member, and wherein the slider pin (110, 210) progressively displaces the pull wire away from the straight line (116, 216) to thereby also generate a presence of the flexure force on the body of the stiffening member and cause the body of the stiffening member to flex inwardly on the first side to thereby facilitate the electrode lead in conforming to a shape of the cochlea of the patient.

13. The electrode lead insertion assembly of claim 12, wherein the track (112, 212, 700, 800) includes a plurality of discrete pin advancement features (802, 806) each configured to haptically indicate, to the user of the pull wire displacement assembly (100, 200) applying the force to the finger slider (608), when the slider pin (110, 210) has advanced along the trajectory (114, 214, 702, 706, 710, 712) from one pin advancement feature to another pin advancement feature in the plurality of discrete pin advancement features.

14. The electrode lead insertion assembly of claim 13, wherein the discrete pin advancement features (802, 806) are disposed along the track (800) at unequal intervals and are each further configured to retain the slider pin (110, 210) in place when the user ceases applying the force to the finger slider (608).

15. The electrode lead insertion assembly of claim 12, wherein the trajectory (702, 706) along which the track (700) is configured to direct the slider pin (110, 210) includes a plurality of linear segments (704-1, 704-2, 708-1, 708-2) each having different slopes,

## Patentansprüche

1. Zugdrahtverlagerungsbaugruppe mit:
einem Gehäuse (102, 202), einer proximalen Drahtbefestigung (104, 204), die an einer proximalen Position entlang des Gehäuses angeordnet ist und ausgebildet ist, um einen Zugdraht (106, 206), der sich entlang des Gehäuses und über ein distales Ende des Gehäuses hinaus erstreckt, statisch zu befestigen;
einer distalen Drahtbefestigung (108, 308), die an einer distalen Position entlang des Gehäuses angeordnet ist und ausgebildet ist, um den Zugdraht in einer Weise gleitend zu fixieren, die es dem Zugdraht erlaubt, sich durch die distale Drahtbefestigung entlang einer Längsachse des Zugdrahts hindurch zu erstrecken, während eine radiale Verlagerung des Zugdrahts an der distalen Drahtbefestigung im Wesentlichen beschränkt wird;
einem Gleitstift (110, 210), der innerhalb des Gehäuses angeordnet ist; und
einer Führungsbahn (112, 212, 700, 800), die innerhalb des Gehäuses angeordnet und entlang welcher der Gleitstift als Reaktion auf eine auf dem Gleitstift angewandte Gleitkraft gleiten kann, wobei die Führungsbahn ausgebildet ist, um den Gleitstift entlang einer Bahnkurve (114, 214, 702, 706, 710, 712) zu führen, **dadurch gekennzeichnet, dass** die Bahnkurve eine gerade Linie (116, 216) zwischen der proximalen und der distalen Drahtbefestigung so kreuzt, dass, wenn der Gleitstift als Reaktion auf die Anwendung der Gleitkraft in einer ersten Richtung entlang der Bahnkurve gleitet, der Gleitstift den Zugdraht progressiv von der geraden Linie weg verlagert, um dadurch einen distalen Abschnitt (120, 220) des Zugdrahts zu dem Gehäuse hin durch die distale Drahtbefestigung hindurch zu ziehen.

2. Zugdrahtverlagerungsbaugruppe gemäß Anspruch 1, wobei sich der Zugdraht (106, 206) jenseits des distalen Endes des Gehäuses (102, 202) entlang eines Versteifungsbauteils (520) erstreckt, welches ausgebildet ist, um ein Einführen einer Elektrodenzuleitung (310, 502) in eine Cochlea eines Patienten zu ermöglichen und einen langgestreckten Körper (522) aufweist, der eine erste Seite sowie eine zweite Seite gegenüberliegend zu der ersten Seite aufweist und ausgebildet ist, um sich mit
einem Abschnitt der Elektrodenzuleitung entlang einer Länger der Elektrodenzuleitung zu integrieren, um den Abschnitt der Elektrodenzuleitung in einer im Wesentlichen linearen Konfiguration zu halten, wenn keine Biegekraft auf den Körper wirkt, wobei die erste Seite ausgebildet ist, um näher bei den Elektroden (312, 512) zu liegen als die zweite Seite, während der Körper mit der Elektrodenzuleitung integriert ist; wobei der distale Abschnitt (120, 220) des Zugdrahts mit einer distalen Spitze des Versteifungsbauteil gekoppelt ist; wobei der Zugdraht eine Biegekraft auf den Körper des Versteifungsbauteils erzeugt, wenn der Gleitstift den Zugdraht progressiv verlagert, um dadurch den distalen Abschnitt des Zugdrahts zu dem Gehäuse hinzuziehen; und
wobei die Biegekraft den Körper des Versteifungsbauteils veranlasst, sich nach innen zu der ersten Seite zu biegen, um dadurch es der Elektrodenzuleitung zu erleichtern, sich an eine Form der Cochlea (400) des Patienten anzugleichen.

3. Zugdrahtverlagerungsbaugruppe gemäß Anspruch 2, wobei das Versteifungsbauteil (520) ferner versehen ist mit:
einer Mehrzahl von Kompressionsschlitzen (524), die entlang der ersten Seite des Körpers verteilt sind und ausgebildet sind, um dem Körper (522) bei Anwesenheit der Biegekraft so vorzuspannen, dass er sich nach innen zu der ersten Seite hin biegt; und
einer Mehrzahl von Zugentlastungsschlitzen (526), die entlang der zweiten Seite des Körpers angeordnet und ausgebildet sind, um bei Anwesenheit der Biegekraft zu expandieren, um die Mehrzahl von Kompressionsschlitzen beim Vorspannen des Körpers zum Biegen nach innen zu der ersten Seite hin zu unterstützen.

4. Zugdrahtverlagerungsbaugruppe gemäß Anspruch 2, wobei:
die Zugdrahtverlagerungsbaugruppe (100, 200) ausgebildet ist, um das Ziehen eines distalen Abschnitts eines zusätzlichen Zugdrahts zu dem Gehäuse (102, 202) hin zu ermöglichen, wobei der distale Abschnitt des zusätzlichen Zugdrahts ferner mit der distalen Spitze des Versteifungsbauteils (520) in einer solchen Weise gekoppelt ist, dass es dem zusätzlichen Zugdraht erlaubt wird, eine Artikulationskraft auf den Körper (522) des Versteifungsbauteils auszuüben, wenn der zusätzliche Zugdraht zu dem Gehäuse hin gezogen wird; und
wobei die Artikulationskraft den Körper des Versteifungsbauteils veranlasst, sich entlang einer Längsachse des Versteifungsbauteils zu verwinden, um es dadurch der Elektrodenzuleitung (310, 502) zu ermöglichen, sich an die Form der Cochlea (400) anzupassen.

5. Zugdrahtverlagerungsbaugruppe gemäß Anspruch 1, wobei die Führungsbahn (112, 212, 700, 800) eine Mehrzahl von diskreten Stiftvorschubmerkmalen (802, 806) aufweist, die jeweils ausgebildet sind, um dem Nutzer der Zugdrahtverlagerungsbaugruppe, welcher die Gleitkraft auf den Gleitstift (110, 210) ausübt, haptisch anzuzeigen, wenn der Gleitstift entlang der Bahnkurve (114, 214, 702, 706, 710, 712) von einem Stiftvorschubmerkmal zu einem anderen Stiftvorschubmerkmal in der Mehrzahl von diskreten Stiftvorschubmerkmalen vorgeschoben wurde.

6. Zugdrahtverlagerungsbaugruppe gemäß Anspruch 5, wobei die diskreten Stiftvorschubmerkmale (802, 806) entlang der Führungsbahn (112, 212, 700, 800) in ungleichen Intervallen angeordnet sind.

7. Zugdrahtverlagerungsbaugruppe gemäß Anspruch 5, wobei die diskreten Stiftvorschubmerkmale (802, 806) jeweils ferner ausgebildet sind, um den Gleitstift (110, 210) an Ort und Stelle zu halten, wenn der Nutzer die Anwendung der Gleitkraft auf den Gleitstift beendet.

8. Zugdrahtverlagerungsbaugruppe gemäß Anspruch 1, wobei die Bahnkurve (702, 706), entlang welcher die Führungsbahn (700) den Gleitstift (110, 210) führt, eine Mehrzahl von linearen Segmenten (704-1, 704-2, 708-1, 708-2) aufweist, die jeweils unterschiedliche Steigungen aufweisen.

9. Zugdrahtverlagerungsbaugruppe gemäß Anspruch 1, wobei die Bahnkurve (710, 712), entlang welcher die Führungsbahn (700) den Gleitstift (110, 210) führt, eine nicht-lineare Kurve beinhaltet.

10. Zugdrahtverlagerungsbaugruppe gemäß Anspruch 1, ferner versehen mit einem Fingergleiter (608), der ausgebildet ist, um:
als Reaktion auf eine von einem Nutzer der Zugdrahtverlagerungsbaugruppe (100, 200) auf den Fingergleiter ausgeübten Kraft entlang des Gehäuses (102, 202) zu gleiten; und
die Gleitkraft auf den Gleitstift (110, 210) anzuwenden, indem die auf den Fingergleiter angewendete Kraft mittels eines physikalischen Kontakts mit dem Gleitstift auf den Gleitstift übertragen wird.

11. Zugdrahtverlagerungsbaugruppe gemäß Anspruch 1, wobei die Führungsbahn (112, 212, 700, 800) ferner ausgebildet ist, um den Gleitstift (110, 210) entlang der Bahnkurve so zu führen, dass, wenn der Gleitstift als Reaktion auf das Anwenden einer Gleitkraft in einer zweiten Richtung entgegengesetzt zu der ersten Richtung gleitet, der Gleitstift es dem Zugdraht (106, 206) progressiv erlaubt, sich an die gerade Linie (116, 216) anzugleichen, um es dadurch dem distalen Abschnitt (120, 220) des Zugdrahts zu erlauben, sich durch die distale Drahtbefestigung (108, 208) hindurch von dem Gehäuse weg zu bewegen.

12. Elektrodenzuleitungseinführbaugruppe mit:
einem Versteifungsbauteil (520) zum Ermöglichen eines Einführens einer Elektrodenzuleitung (310, 502) in eine Cochlea (400) eines Patienten, das einen langgestreckten Körper (522) aufweist, welcher eine erste Seite und eine zweite Seite entgegengesetzt zu der ersten Seite aufweist und ausgebildet ist, um sich mit einem Abschnitt der Elektrodenzuleitung entlang einer Länge der Elektrodenzuleitung zu integrieren, um den Abschnitt der Elektrodenzuleitung in einer im Wesentlichen linearen Konfiguration zu halten, wenn keine Biegekraft auf den Körper wirkt, wobei die erste Seite näher an den Elektroden (312, 512) als die zweite Seite liegt, während der Körper mit der Elektrodenzuleitung integriert ist;
sowie der Zugdrahtverlagerungsbaugruppe (100, 200) von Anspruch 10; und
dem Zugdraht (106, 206), wobei sich der Zugdraht entlang des Versteifungsbauteils erstreckt und an einem distalen Abschnitt (120, 220) des Zugdrahts mit einer distalen Spitze des Versteifungsbauteils gekoppelt ist, und wobei der Gleitstift (110, 210) den Zugdraht progressiv weg von der geraden Linie (116, 216) verlagert, um dadurch auch eine Biegekraft auf den Körper des Versteifungsbauteils zu erzeugen und den Körper des Versteifungsbauteils dazu veranlassen, sich auf der ersten Seite nach innen zu biegen, um es dadurch der Elektrodenzuleitung zu ermöglichen, sich an eine Form der Cochlea des Patienten anzugleichen.

13. Elektrodenzuleitungseinführbaugruppe gemäß Anspruch 12, wobei die Führungsbahn (112, 212, 700, 800) eine Mehrzahl von diskreten Stiftvorschubmerkmalen (802, 806) aufweist, die jeweils ausgebildet sind, um dem Nutzer der Zugdrahtverlagerungsbaugruppe (100, 200), welcher die Kraft auf den Fingergleiter (608) ausübt, haptisch anzuzeigen, wenn der Gleitstift (110, 210) entlang der Bahnkurve (114, 214, 702, 706, 710, 712) von einem Stiftvorschubmerkmal zu einem anderen Stiftvorschubmerkmal in der Mehrzahl von diskreten Stiftvorschubmerkmalen vorgeschoben wurde.

14. Elektrodenzuleitungseinführbaugruppe gemäß Anspruch 13, wobei die diskreten Stiftvorschubmerkmale (802, 806) entlang der Führungsbahn (800) in ungleichen Intervallen angeordnet und ferner ausgebildet sind, um den Gleitstift (110, 210) an Ort und Stelle zu halten, wenn der Nutzer das Anwenden der Kraft auf den Fingergleiter (608) beendet.

15. Elektrodenzuleitungseinführbaugruppe gemäß Anspruch 12, wobei die Bahnkurve (702, 706), entlang welcher die Führungsbahn (702) den Stift (110, 210) führt, eine Mehrzahl von linearen Segmenten (704-1, 704-2, 708-1, 708-2) aufweist, die jeweils unterschiedliche Steigungen haben.

## Revendications

1. Ensemble de déplacement d'un fil de traction comprenant :
un boîtier (102, 202) ;
un support de fil proximal (104, 204) disposé au niveau d'une position proximale le long du boîtier et conçu pour se fixer de manière statique à un fil de traction (106, 206) qui s'étend le long du boîtier et au-delà d'une extrémité distale du boîtier ;
un support de fil distal (108, 208) disposé au niveau d'une position distale le long du boîtier et conçu pour se fixer de manière coulissante au fil de traction d'une manière qui permet que le fil de traction traverse le support de fil distal le long d'un axe longitudinal du fil de traction tout en limitant sensiblement un déplacement radial du fil de traction au niveau du support de fil distal ;
une broche coulissante (110, 210) disposée à l'intérieur du boîtier ; et
une piste (112, 212, 700, 800) disposée à l'intérieur du boîtier et le long de laquelle la broche coulissante est conçue pour coulisser en réponse à une force de coulissement appliquée à la broche coulissante, la piste étant conçue pour diriger la broche coulissante le long d'une trajectoire (114, 214, 702, 706, 710, 712), **caractérisé en ce que** la trajectoire croise une ligne droite (116, 216) entre les supports de fil proximal et distal de telle sorte que, lorsque la broche coulissante coulisse le long de la trajectoire en réponse à une application de la force de coulissement dans une première direction, la broche coulissante déplace progressivement le fil de traction à l'écart de la ligne droite pour tirer de ce fait une partie distale (120, 220) du fil de traction vers le boîtier à travers le support de fil distal.

2. Ensemble de déplacement de fil de traction selon la revendication 1, dans lequel :
au-delà de l'extrémité distale du boîtier (102, 202), le fil de traction (106, 206) s'étend le long d'un élément de raidissement (520) conçu pour faciliter une insertion d'un fil d'électrode (310, 502) dans une cochlée d'un patient, l'élément de raidissement comprenant un corps allongé (522) qui a un premier côté et un second côté opposé au premier côté et qui est conçu pour s'intégrer à une partie du fil d'électrode sur une longueur du fil d'électrode de sorte à maintenir la partie du fil d'électrode dans une configuration sensiblement linéaire en l'absence d'une force de flexion sur le corps, le premier côté étant conçu pour être plus proche des électrodes (312, 512) que le second côté lorsque le corps est intégré au fil d'électrode ;
la partie distale (120, 220) du fil de traction est accouplée à une pointe distale de l'élément de raidissement ;
le fil de traction génère une présence de la force de flexion sur le corps de l'élément de raidissement lorsque la broche coulissante déplace progressivement le fil de traction pour tirer de ce fait la partie distale du fil de traction vers le boîtier ; et
la présence de la force de flexion amène le corps de l'élément de raidissement à fléchir vers l'intérieur sur le premier côté pour aider de ce fait le fil d'électrode à épouser la forme de la cochlée (400) du patient.

3. Ensemble de déplacement de fil de traction selon la revendication 2, dans lequel l'élément de raidissement (520) comprend en outre :
une pluralité de fentes de compression (524) réparties le long du premier côté du corps, la pluralité de fentes de compression étant conçue pour se comprimer, en présence de la force de flexion, de sorte à solliciter le corps (522) pour qu'il fléchisse vers l'intérieur sur le premier côté ; et
une pluralité de fentes d'allégement de contraintes (526) réparties le long du second côté du corps,
la pluralité de fentes d'allégement de contraintes étant conçue pour se dilater, en présence de la force de flexion, de sorte à compléter la pluralité de fentes de compression en sollicitant le corps pour qu'il fléchisse vers l'intérieur sur le premier côté.

4. Ensemble de déplacement de fil de traction selon la revendication 2, dans lequel :
l'ensemble de déplacement de fil de traction (100, 200) est conçu pour faciliter la traction d'une partie distale d'un fil de traction supplémentaire vers le boîtier (102, 202) ;
la partie distale du fil de traction supplémentaire est en outre accouplée avec la pointe distale de l'élément de raidissement (520) d'une manière qui permet que le fil de traction supplémentaire génère une présence d'une force d'articulation sur le corps (522) de l'élément de raidissement lorsque le fil de traction supplémentaire est tiré vers le boîtier ; et
la présence de la force d'articulation amène le corps de l'élément de raidissement à se tordre le long d'un axe longitudinal de l'élément de raidissement pour aider encore de ce fait fil d'électrode (310, 502) à épouser la forme de la cochlée (400).

5. Ensemble de déplacement de fil de traction selon la revendication 1, dans lequel la piste (112, 212, 700, 800) comprend une pluralité de caractéristiques discrètes d'avancement de broche (802, 806), chacune conçue pour indiquer de manière haptique, à un utilisateur de l'ensemble de déplacement de fil de traction appliquant la force de coulissement à la broche coulissante (110, 210), le moment où la broche coulissante a avancé le long de la trajectoire (114, 214, 702, 706, 710, 712) d'une caractéristique d'avancement de broche à une autre caractéristique d'avancement de broche dans la pluralité de caractéristiques discrètes d'avancement de broche.

6. Ensemble de déplacement de fil de traction selon la revendication 5, dans lequel les caractéristiques discrètes d'avancement de broche (802, 806) sont disposées le long de la piste (112, 212, 700, 800) à des intervalles inégaux.

7. Ensemble de déplacement de fil de traction selon la revendication 5, dans lequel les caractéristiques discrètes d'avancement de broche (802, 806) sont chacune conçues en outre pour retenir la broche coulissante (110, 210) en place lorsque l'utilisateur cesse d'appliquer la force de coulissement à la broche coulissante.

8. Ensemble de déplacement de fil de traction selon la revendication 1, dans lequel la trajectoire (702, 706) le long de laquelle la piste (700) est conçue pour diriger la broche coulissante (110, 210) comprend une pluralité de segments linéaires (704-1, 704-2, 708-1, 708-2) ayant chacun des pentes différentes.

9. Ensemble de déplacement de fil de traction selon la revendication 1, dans lequel la trajectoire (710, 712) le long de laquelle la piste (700) est conçue pour diriger la broche coulissante (110, 210) comprend une courbe non linéaire.

10. Ensemble de déplacement de fil de traction selon la revendication 1, comprenant en outre un curseur de doigt (608) conçu pour :
coulisser le long du boîtier (102, 202) en réponse à une force appliquée au curseur de doigt par un utilisateur de l'ensemble de déplacement de fil de traction (100, 200) ; et
appliquer la force de coulissement à la broche coulissante (110, 210) en transférant la force appliquée au curseur de doigt à la broche coulissante au moyen d'un contact physique avec la broche coulissante.

11. Ensemble de déplacement de fil de traction selon la revendication 1, dans lequel la piste (112, 212, 700, 800) est en outre conçue pour diriger la broche coulissante (110, 210) le long de la trajectoire de telle sorte que, lorsque la broche coulissante coulisse le long de la trajectoire (114, 214, 702, 706, 710, 712) en réponse à une application de la force de coulissement dans une seconde direction opposée à la première direction, la broche coulissante permette progressivement que le fil de traction (106, 206) épouse la forme de la ligne droite (116, 216) pour permettre de ce fait que la partie distale (120, 220) du fil de traction s'éloigne du boîtier à travers le support de fil distal (108, 208).

12. Ensemble d'insertion de fil d'électrode comprenant :
un élément de raidissement (520) conçu pour faciliter une insertion d'un fil d'électrode (310, 502) dans une cochlée (400) d'un patient, l'élément de raidissement comprenant un corps allongé (522) qui a un premier côté et un second côté opposé au premier côté et qui est conçu pour s'intégrer à une partie du fil d'électrode sur une longueur du fil d'électrode de sorte à maintenir la partie du fil d'électrode dans une configuration sensiblement linéaire en l'absence d'une force de flexion sur le corps, le premier côté étant conçu pour être plus proche des électrodes (312, 512) que le second côté pendant que le corps est intégré au fil d'électrode ;
l'ensemble de déplacement de fil de traction (100, 200) selon la revendication 10 ; et
le fil de traction (106, 206), dans lequel le fil de traction s'étend le long de l'élément de raidissement et est accouplé, au niveau d'une partie distale (120, 220) du fil de traction, avec une pointe distale de l'élément de raidissement, et dans lequel la broche coulissante (110, 210) déplace progressivement le fil de traction à l'écart de la ligne droite (116, 216) pour générer ainsi également une présence de la force de flexion sur le corps de l'élément de raidissement et amener le corps de l'élément de raidissement à fléchir vers l'intérieur sur le premier côté pour aider ainsi le fil d'électrode à épouser la forme de la cochlée du patient.

13. Ensemble d'insertion de fil d'électrode selon la revendication 12, dans lequel la piste (112, 212, 700, 800) comprend une pluralité de caractéristiques discrètes d'avancement de broche (802, 806) chacune conçue pour indiquer de manière haptique, à l'utilisateur de l'ensemble de déplacement de fil de traction (100, 200) appliquant la force au curseur de doigt (608), le moment où la broche coulissante (110, 210) a avancé le long de la trajectoire (114, 214, 702, 706, 710, 712) d'une caractéristique d'avancement de broche à une autre caractéristique d'avancement de broche dans la pluralité de caractéristiques discrètes d'avancement de broche.

14. Ensemble d'insertion de fil d'électrode selon la revendication 13, dans lequel les caractéristiques discrètes d'avancement de broche (802, 806) sont disposées le long de la piste (800) à des intervalles inégaux et sont chacune en outre conçues pour retenir la broche coulissante (110, 210) en place lorsque l'utilisateur cesse d'appliquer la force sur le curseur de doigt (608) .

15. Ensemble d'insertion de fil d'électrode selon la revendication 12, dans lequel la trajectoire (702, 706) le long de laquelle la piste (700) est conçue pour diriger la broche coulissante (110, 210) comprend une pluralité de segments linéaires (704-1, 704-2, 708-1, 708-2) ayant chacun des pentes différentes.
